(19) Europäisches Patentamt
European Patent Office
Office européen des brevets

(11) Publication number: **0 221 019 B1**

(12) **EUROPEAN PATENT SPECIFICATION**

(45) Date of publication of patent specification: **05.01.94**

(51) Int. Cl.5: **C07K 5/00**, C07D 207/28, A61K 37/02, A61K 31/195, A61K 31/215, A61K 31/325

(21) Application number: **86810444.9**

(22) Date of filing: **08.10.86**

(54) **Peptides.**

(30) Priority: **14.10.85 JP 229648/85**

(43) Date of publication of application:
**06.05.87 Bulletin 87/19**

(45) Publication of the grant of the patent:
**05.01.94 Bulletin 94/01**

(84) Designated Contracting States:
**AT BE CH DE ES FR GB GR IT LI LU NL SE**

(56) References cited:
**EP-A- 0 054 862**
**EP-A- 0 077 724**
**EP-A- 0 154 234**
**FR-A- 2 546 517**
**US-A- 4 316 892**
**US-A- 4 634 716**

**Chemical Abstracts, vol. 57, No.7 October 1, 1962, column 8859e, Columbus Ohio, US; J M Clayton et al.: "Isolation of Gamma-L-glutamyl-Beta-alanine from iris bulbs"**

(73) Proprietor: **NIPPON ZOKI PHARMACEUTICAL CO. LTD.**
**10, 2-chome Hiranomachi**
**Higashi-ku**
**Osaka(JP)**

(72) Inventor: **Ienaga, Kazuharu Inst. Bio-Active-Science**
**Nippon Zoki Pharmaceutical Co. Ltd.**
**442-1, Kinashi**
**Yashiro-cho Katoh-gun Hyogo(JP)**
Inventor: **Hagashiura, Kunihiko Inst. Bio-Active Science**
**Nippon Zoki Pharmaceutical Co. Ltd.**
**442-1, Kinashi**
**Yashiro-cho Katoh-gun Hyogo(JP)**

(74) Representative: **Kerr, Andrew**
**Postfach 122**
**Finkelerweg 44**
**CH-4144 Arlesheim BL (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

Chemical Abstracts, vol.65, No. 8, October 10, 1966, column 12281g, Columbus, Ohio, US; IG Orlov et al.:"Kinetics of the hydrolosis of peptides in alkalis. V. The hydrolosis of glycl-DL-alpha-alamine and glycl-Beta-alanine in aqueous solutions of KOH"

Chemical Abstracts, vol.63, No. 11, November 22, 1965, column 15136f Columbus, Ohio, US; P Olesen Larsen: "Amino acids and Beta-glutamyl derivatives in seeds of Lunaria annua II"

Chemical Abstracts, vol. 72, No.15, April 13, 1970, page 365, abstract 78713r, Columbus Ohio, US

Chemical Abstracts, vol. 78, No.8, February 26, 1973, page 362, abstract 48910j, Columbus, Ohio, US; A P Brunetti et al.:"Thermodynamics of ion association. XXIII. Copper, zinc, and cadmium complexes of some dipeptides

Chemical Abstracts, vol. 81, no.5, August 5, 1974, page 457, abstract 25927t, Columbus, Ohio, US; A L Poznyak et al.: "Free radicals during photooxidation of amino acids and dipeptides sensitized by iron (III) complexes

Chemical Abstracts, vol. 99, No. 19, November 7, 1983, page 657, abstract 158828e, Columbus, Ohio, US; O Agostini et al.: "Synthesis of five N-derivatives of 1, 2, 3, 4-tetrahydroisoquinoline and three N-terminal histidine peptides as potential gastric histidine decarboxylase inhibitors"

Journal of the Chemical Society, Part V, 1965, pages 7305-7312, London, GB; A P Fosker et al.: "L-Glutamyl-Gamma-aminobutyric acid and related compounds"

Chemical Abstracts, vol. 99, No 5, August 1, 1983, page 100, abstract 33528a, Columbus, Ohio, US; M J Peet et al.: "Antagonists of synaptic and amino acid excitation of neurons in the cat spinal cord"

Chemical Abstracts, vol. 100, No.1, January 2, 1984, page 625, abstract 7162w, Columbus, Ohio, US

Chemical Abstracts, vol. 102, 1985, page 22, astract 72407k, Columbus Ohio, US; A W Jones et al.: "Structure-activity relations of dipeptide antagonists of excitatory amino acids"

Chemical Abstracts, vol. 104, No. 17, April 28, 1986, page 753, abstract 149422n, Columbus, Ohio, US.

Chemical Abstracts, vol. 76, No.23, June 5 1972, page 33, abstract 135726f, Columbus Ohio, US; E A Bendikov et al.: "Influence of Gamma-aminobutyric acid, nicotinoyl-Gamma-aminobutyric and its ethyl esters on central processes of vasomotor reflex formation"

Chemical Abstracts, vol. 94, No.17, April 27, 1981, page 800, abstract 140138s, Columbus, Ohio, US; L A Zhmurenko et al.: "Synthesis and pharmacological study of N-(2-substituted nicotinoyl) amine acids"

Chemical Abstracts, vol. 105, No. 25, December 22, 1986, page 865, abstract 227325v, Columbus, Ohio, US

CHEMICAL ABSTRACTS, Vol. 84, No. 17, April 26, 1976, page 591, abstract 122346x, Columbus, Ohio, US

## Description

The present invention relates to peptides, pharmaceutically acceptable salts and complexes thereof, and pharmaceutical compositions containing peptides as active ingredients.

Various peptides and derivatives thereof have been synthesised, and their pharmaceutical activity studied. Certain di- and tripeptides have been found to have pharmaceutical, notably neurotropic activity. Some of these are known per se or for non-pharmaceutical applications. Others are novel.

It is an object of the present invention to provide peptides which are useful as medicines and/or as intermediates for the synthesis of further compounds. The invention is inclusive of a process for the preparation of the novel peptides.

Chemical Abstracts (CA), 1974, 4,25927t, CA 99, 1983, 158828e, CA 78, 1973, 48910j, CA 72, 1970, 78713x, CA 63, 1965, 15136f, CA 65, 1966, 12281g, CA 57, 1962, 8859e, EP-A-54862, FR-A-2 546 517, J. Chem. Soc., part V, 1965, pp 7305-7312, CA 84, 1976, 122346x, CA 99, 1983, 33528a, CA 100, 1984, 7162w, CA 104, 1986, 149422n, and CA 105, 1986, 227325v describe various dipeptides having carboxy-terminal acid groups with less than 3 (non-carboxy) carbon atoms and/or glycine (Gly), glutamic acid (Glu), 5-Hydroxytryptophan (5-HTP) histidine (His) or phenylalanine (Phe) at the amino terminal.

In general, pharmaceutical activity is not disclosed. EP-A- 54862 describes inhibition of a specific opiate receptor; FR-A-2546517 describes A-terminus 5-HTP dipeptides having activity against disorders of the serotoninergetic system; The J. Chem. Soc. reference (above) describes A-terminus Glu dipeptides (and certain derivatives) having activity associated with the central inhibitory process in mammals; CA 84 (above) describes Pyr-β-Ala and Pyr-GABA as having antidepressive activity;
CA 99 (above) describes D-β-Asp-β-Ala and y-Glu-Gly as having some activity in the excitation of neurons in the cat spinal chord, and CA 105, 1986, 227325v describes analgesic and anaesthetic activity in certain substituted dipeptides.

CA 100, 1984, 7162w (above) also discloses as flavouring agents Phe-GABA, Phe-GABOB and Tyr-GABOB.

According to one aspect of the invention there is provided a pharmaceutical composition containing as an active ingredient a peptide having the formula (I)

A-X-NH-Y-CO-R

where:

A is hydrogen, an amino protecting group, an acidic amino acid residue, or an acidic amino acid residue having a protecting group, and wherein:

(i) when A is hydrogen or an amino protecting group: X is selected from Ala, Val, Leu, Ile, Pro, Ser, Thr, Tyr, Hyp, Asp, D-Asp, β-Asp, D-β-Asp, Phe and Pyr, with or without a protecting group;
Y is straight $C_{3-6}$ alkylene or hydroxyalkylene, and
R is a hydroxy group or a carboxy-protecting group;
provided that Pyr-GABA is excluded;

(ii) when A is an acidic amino acid residue or an acidic amino acid having a protecting group:
X is an amino acid residue with or without a protecting group;
Y is straight $C_{2-6}$ alkylene or hydroxyalkylene, and
R is a hydroxy group or a carboxy-protecting group;
or a pharmaceutically acceptable salt or complex thereof.

According to another aspect of the invention thereis provided a peptide having the formula (I)

A-X-NH-Y-CO-R

where:

A is hydrogen, an amino protecting group, an acidic amino acid residue, or an acidic amino acid residue having a protecting group, and wherein:

(i) when A is hydrogen or an amino protecting group:
X is selected from Ala, Val, Leu, Ile, Pro, Ser, Thr, Tyr, Hyp, Asp, D-Asp, β-Asp, D-β-Asp, and Pyr, with or without a protecting group;
Y is straight $C_{3-6}$ alkylene or hydroxyalkylene, and
R is a hydroxy group or a carboxy-protecting group;
provided that Pyr-GABA and Tyr-GABOB are excluded;

(ii) when A is an acidic amino acid residue or an acidic amino acid having a protecting group:
X is an amino acid residue with or without a protecting group;
Y is straight $C_{2-6}$ alkylene or hydroxyalkylene, and
R is a hydroxy group or a carboxy-protecting group;
or a pharmaceutically acceptable salt or complex thereof.

The peptides and amino acids are herein expressed by abbreviations adopted by IUPAC and IUC or by abbreviations commonly used in the art to which the invention pertains.

For example, the following abbreviations are used:-

| | | | |
|---|---|---|---|
| Gly | glycine | Ala | alanine |
| Val | valine | Leu | leucine |
| Ile | isoleucine | Phe | phenylalanine |
| Pro | proline | Ser | serine |
| Thr | threonine | Tyr | tyrosine |
| Lys | lysine | Hyp | hydroxyproline |
| His | histidine | Asp | aspartic acid |
| Arg | arginine | Glu | glutamic acid |
| Trp | tryptophan | 5-HTP | 5-hydroxytryptophan |
| Cys | cysteine | Met | methionine |
| β-Ala | β-alanine | Pyr | pyroglutamic acid |
| GABA | γ-aminobutyric acid | | |
| GABOB | γ-amino-β-hydroxybutyric acid | | |
| EACA | ε-aminocaproic acid | | |

The amino acid residues may be any of the D-isomer, L-isomer or DL-isomer.

As the amino-protecting group, any of those employed in conventional peptide synthesis can be used, i.e. a lower alkoxycarbonyl group such as t-butoxycarbonyl or t-pentoxycarbonyl, an aralkyloxycarbonyl group such as benzyloxycarbonyl, or a substituted aralkyloxycarbonyl group such as o-chlorobenzyloxycarbonyl, p-nitrobenzyloxycarbonyl, or p-methoxybenzyloxycarbonyl.

Acidic amino acid residues may include for example α-Glu, γ-Glu, α-Asp, or β-Asp. As the protecting group of a terminal amino group of an acid amino acid residue the above mentioned amino-protecting groups can be employed. As the protecting group for a free carboxy group of an acid amino acid residue there can be used any of those employed in conventional peptide synthesis, i.e. an aralkyloxy group such as benzyloxy, or a substituted aralkyloxy group such as p-methoxybenzyloxy etc. or 4-pyridyloxy.

X is an amino acid residue which may optionally have one or more protecting groups, e.g. γ-Glu, β-Asp or an amino acid which constitutes protein, and is preferably Gly, Glu, Lys, Tyr, Asp, Phe, Ile, Ala, Pro, Leu, Hyp, Val, His, Arg, Ser, Thr, Pyr, Trp, 5-HTP, Cys, Met, γ-Glu, β-Asp. As a protecting group of the amino acid there my be used any of those conventionally employed in peptide synthesis, i.e protecting groups for amino or carboxy groups as mentioned above, or a hydroxy-protecting group such as a benzyl group.

In the case of tripeptides (Formula I(ii) above) Y is a straight alkylene group having from 2 to 6 carbon atoms, e.g. ethylene, propylene, butylene, pentylene, hexylene, or a straight $C_{2-6}$ alkylene group as above having one or more hydroxy groups, preferably one hydroxy group. In the case of dipeptides (Formula I(i) above) Y has at least three carbon atoms but is otherwise the same. The amino acid residue at the C-terminal containing the Y group is preferably GABA, GABOB or EACA, but may also be β-Ala in the case of tripeptides.

In compositions according to the invention, preferred peptides include the following:-

Ala-GABA, Val-GABA, Leu-GABA, Ile-GABA, Phe-GABA*, Pro-GABA, Ser-GABA, Thr-GABA, Tyr-GABA, Hyp-GABA, Asp-GABA, β-Asp-GABA, D-Asp-GABA, D-β-Asp-GABA,
Ala-GABOB, Val-GABOB, Leu-GABOB, Ile-GABOB, Phe-GABOB*, Pro-GABOB, Ser-GABOB, Thr-GABOB, Tyr-GABOB*, Hyp-GABOB, Asp-GABOB, β-Asp-GABOB, D-Asp-GABOB, D-β-Asp-GABOB, Pyr-GABOB, Ala-EACA, Val-EACA, Leu-EACA, Ile-EACA, Phe-EACA, Pro-EACA, Ser-EACA, Thr-EACA, Hyp-EACA, Asp-EACA, β-Asp-EACA, D-Asp-EACA, D-β-Asp-EACA, Pyr-EACA,
γ-Glu-Gly-GABA, γ-Glu-Ala-GABA, γ-Glu-Val-GABA,
γ-Glu-Leu-GABA, γ-Glu-Ile-GABA, γ-Glu-Phe-GABA,
γ-Glu-Pro-GABA, γ-Glu-Ser-GABA, γ-Glu-Thr-GABA,
γ-Glu-Tyr-GABA, γ-Glu-Hyp-GABA, γ-Glu-Lys-GABA,
γ-Glu-His-GABA, γ-Glu-Arg-GABA, γ-Glu-Asp-GABA,

γ-Glu-Glu-GABA, γ-Glu-β-Asp-GABA,
γ-Glu-γ-Glu-GABA, γ-Glu-D-Asp-GABA,
γ-Glu-D-β-Asp-GABA, γ-Glu-Trp-GABA,
γ-Glu-5-HTP-GABA, γ-Glu-Cys-GABA, γ-Glu-Met-GABA,
γ-Glu-Phe-β-Ala, γ-Glu-Phe-GABOB, γ-Glu-Phe-EACA,
Glu-Phe-GABA, Glu-Phe-β-Ala, Glu-Phe-GABOB, Glu-Phe-EACA, Asp-Phe-GABA, Asp-Phe-β-Ala, Asp-Phe-GABOB, Asp-Phe-EACA, β-Asp-Phe-GABA, β-Asp-Phe-β-Ala, β-Asp-Phe-GABOB,
β-Asp-Phe-EACA,
D-Asp-Phe-GABA, D-Asp-Phe-β-Ala, D-Asp-Phe-GABOB,
D-Asp-Phe-EACA,
D-β-Asp-Phe-GABA, D-β-Asp-Phe-β-Ala, D-β-Asp-Phe-GABOB, D-β-Asp-Phe-EACA,
γ-Glu-Pro-β-Ala, γ-Glu-Pro-GABOB, γ-Glu-Pro-EACA, Glu-Pro-GABA, Glu-Pro-β-Ala, Glu-Pro-GABOB, Glu-Pro-EACA, Asp-Pro-GABA, Asp-Pro-β-Ala, Asp-Pro-GABOB, Asp-Pro-EACA, β-Asp-Pro-GABA, β-Asp-Pro-β-Ala, β-Asp-Pro-GABOB, β-Asp-Pro-EACA,
D-Asp-Pro-GABA, D-Asp-Pro-β-Ala, D-Asp-Pro-GABOB, D-Asp-Pro-EACA,
D-β-Asp-Pro-GABA, D-β-Asp-Pro-β-Ala, D-β-Asp-Pro-GABOB, D-β-Asp-Pro-EACA,

However those marked (*) are known and excluded from the novel compounds of the invention.

The peptide compounds of the invention also include pharmaceutically acceptable salts of the compounds of Formula (I) e.g. acid addition salts with inorganic acids such as hydrochloric acid, sulphuric acid, nitric acid, hydrobromic acid, phosphoric acid, perchloric acid, thicyanic acid, and boric acid etc., or with organic acids such as formic acid, acetic acid, haloacetic acid, propionic acid, glycolic acid, citric acid, tartaric acid, succinic acid, gluconic acid, lactic acid, malonic acid, fumaric acid, anthranilic acid, benzoic acid, cinnamic acid, p-toluenesulfonic acid, naphthalenesulfonic acid, sulfanilic acid etc., and salts with metals such as alkali metal, e.g. sodium, potassium, lithium etc., and aluminium etc.

The peptide compounds of the invention may include their metal complexes, such as for example complexes with zinc, nickel, cobalt, copper, iron etc.

These salts and metal complexes can be produced from free peptide compounds in a conventional manner or can be interchanged with each other.

The peptide compounds according to the present invention can be produced by conventional processes in peptide chemistry, and either the solution method or solid phase synthesis can be employed.

The coupling method for forming a peptide bond can include the azide method, active ester method, mixed acid anhydride method, acid chloride method, method employing a coupling reagent etc., and they can be employed either alone or in combination as desired.

As amino acid or peptide used as the starting material or materials in the coupling reaction, there are employed those having an appropriate substituent constituting group conventionally employed in peptide chemistry. Further, any carboxy group or amino groups which do not participate in the reaction can be protected by a known method, or any carboxy group or amino group which participates in the reaction can be activated.

The abbreviations used for the substitutes, reagents etc. are as follows:

| | |
|---|---|
| Z: | Benzyloxycarbonyl |
| Boc: | t-Butoxycarbonyl |
| Bzl: | Benzyl |
| OBzl: | Benzyloxy |
| HONSu: | N-hydroxysuccinimide |
| ONSu: | N-hydroxysuccinimide ester |
| HONB: | N-hydroxy-5-norbornene-2,3-dicarboxyimide |
| ONB: | N-hydroxy-5-norbornene-2,3-dicarboxyimide ester |
| DSC: | Disuccinimidylcarbonate |
| DCC: | Dicyclohexylcarbodiimide |
| DCUrea: | Dicyclohexyl urea |
| WSCD: | 1-ethyl-3-(3-dimethylaminopropyl)-carbodiimide (Water-soluble carbodiimide) |
| DCHA: | Dicyclohexylamine |
| ECF: | Ethyloxycarbonyl chloride |
| NMM: | N-Methylmorpholine |
| TosOH: | p-Toluenesulfonic acid |
| THF: | Tetrahydrofuran |
| TEA: | Triethylamine |
| DMF: | Dimethylformamide |

The peptide compounds of the present invention can be prepared as follows.

I. Preparation of Dipeptide compound

Method I (1):

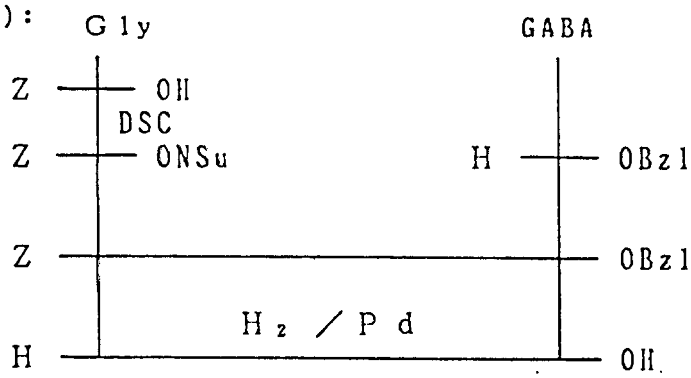


Method I (2):

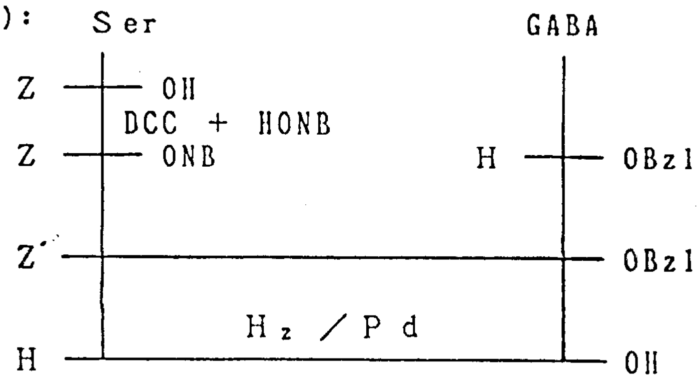

Method I(3):

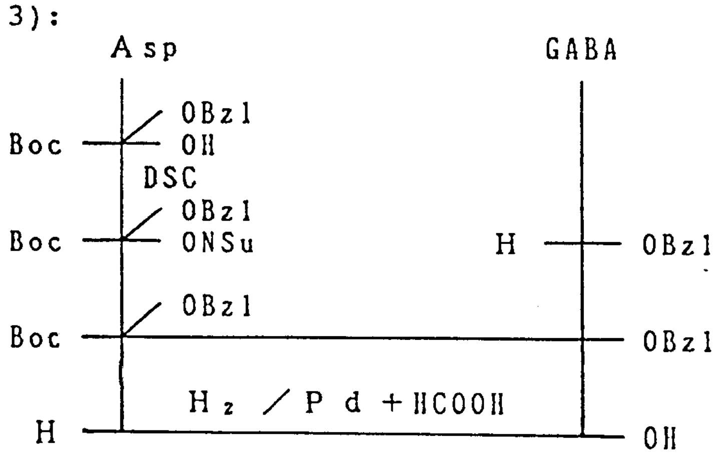


Method I(4):

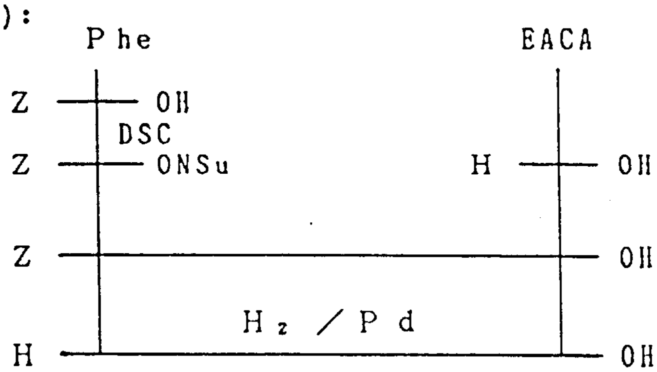


Method I(5):

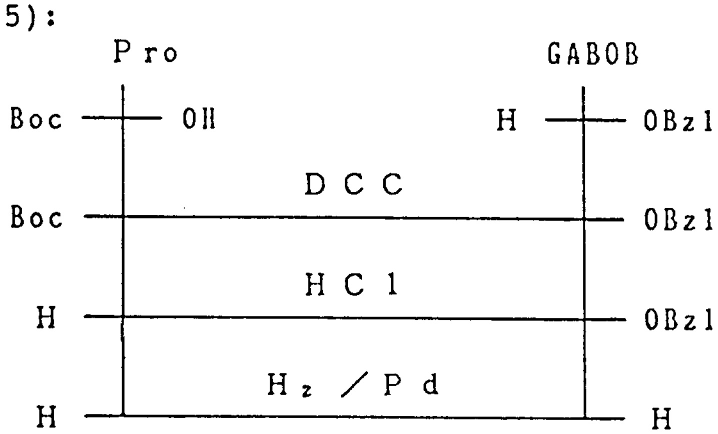

II. Preparation of Tripeptide compound

Method II(1):

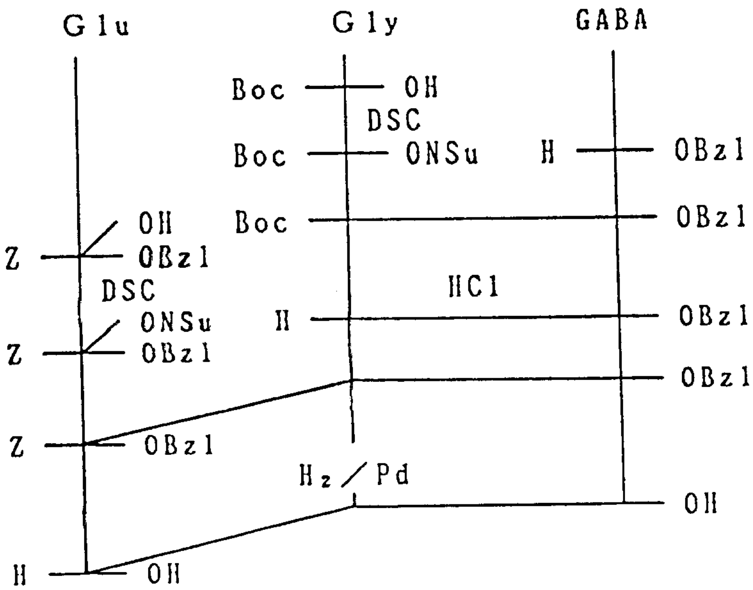


Method II(2):

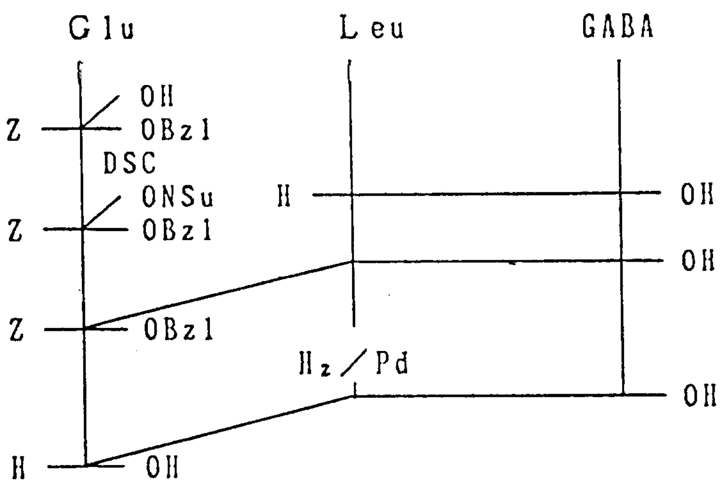

Method II(3):

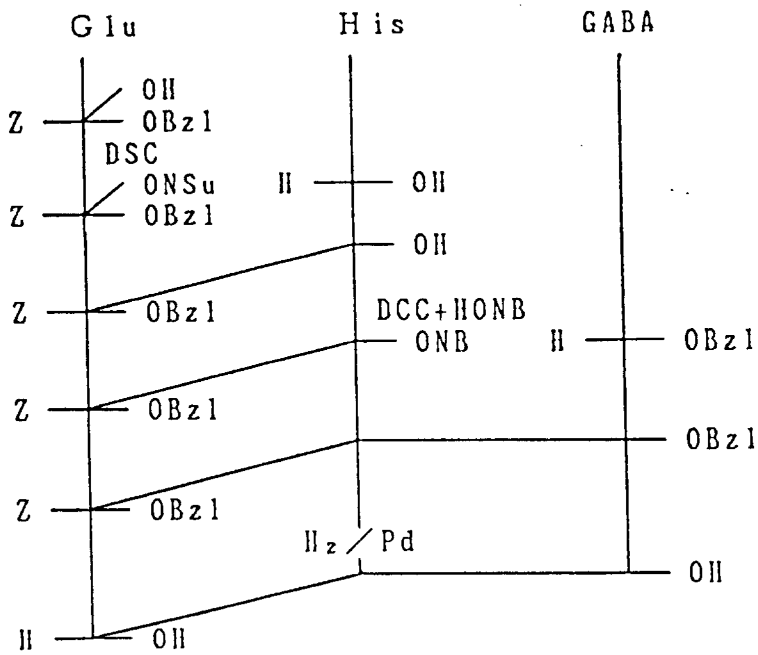


Method II(4):

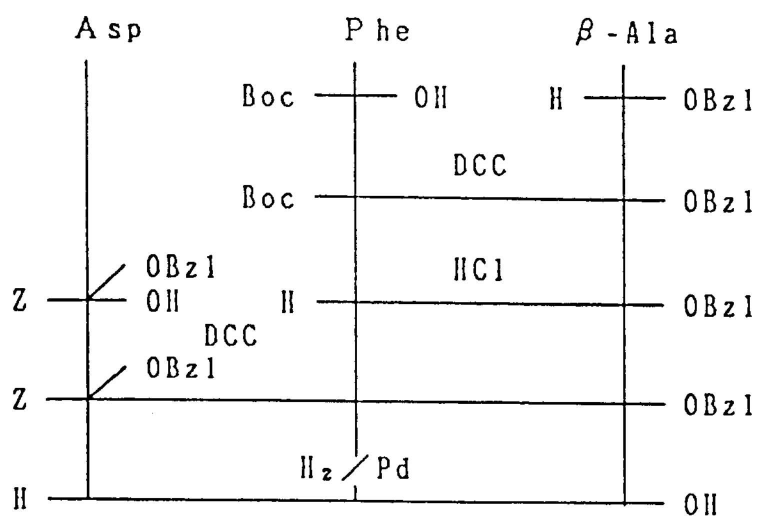

Method II(5):

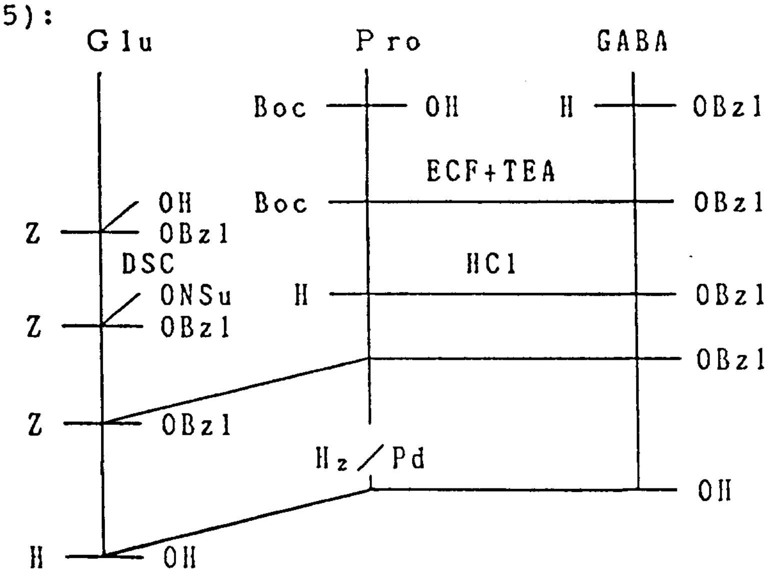


The above scheme illustrates an example of a process for preparing a peptide compound of the present invention. Each of α-, β- or γ-amino acid can be similarly employed as the amino acid in the above schemes.

Method I(1)-(4) and Method II(1)-(3) show methods for preparing the peptide compounds of the invention according to the activated ester method using ONSu or ONB. The ONSu esterification can also be carried out by using both HOSu and DCC instead of DSC.

As the protecting group of an amino group at N-terminal and of the side chain, o-chlorobenzyloxycarbonyl, p-nitrobenzyloxycarbonyl or p-methoxybenzyloxycarbonyl group can be employed as well as benzyloxycarbonyl (Z), and t-pentoxycarbonyl or p-methoxybenzyloxycarbonyl group can be employed as well as t-butoxycarbonyl group (Boc).

As the protecting group of the C-terminal carboxyl group and of the side chain, p-nitrobenzyloxy or 4-pyridyloxy group can be employed as well as the benzyloxy group (OBzl). But it is not necessary to protect the carboxy group according to the reaction Methods I(4), Method II(2) and (3) . The hydroxy group of the side-chain can be protected by a benzyl group etc.

These substituents can be selectively or entirely removed by the conventional method such as catalytic reduction, acidolysis etc. either in the course of the synthetic process of the peptide compound of this invention or in its final stage. Further, if necessary, it is also possible to introduce a substituent which constitutes other peptide compound of the invention by conventional methods.

Method II(1) shows that the amino acids which constitute the peptide compounds of the invention are condensed successively from the C-terminal to the N-terminal. On the other hand, Method II(3) is the peptide-elongating method from N-terminal to C-terminal. Therefore, the peptide compounds of the present invention can be prepared by either method elongating from N- or C-terminal.

Furthermore, instead of condensation of the constituent amino acid, tripeptides of the present invention can be prepared by using the dipeptide of this invention such as Leu-GABA and the like as a starting material [Method II(2)].

DCC can be used not only as an esterifying agent for forming active ester such as ONB etc. but also as a condensing agent directly forming a peptide bond [Method I(5) and Method II(4)]. Water-soluble carbodiimides such as 1-ethyl-3-(3-dimethylaminopropyl)-carbodiimide hydrochloride can also be employed similarly to DCC as a condensing agent.

The peptide compounds of the present invention can be prepared by the mixed acid anhydride method [Method II(5)]. As an acidic component of the mixed acid anhydride, ethyloxycarbonyl chloride etc. can be employed, and triethylamine, N-methylmorpholinon etc. can be employed as a basic component.

The solvent employed in the processes of preparation of the present invention is preferably inert both to starting materials and products, and preferably dissolves both, but suspending condition can also be employed. Solvents or suspending agents which can be used include, for example, tetrahydrofuran, ethyl acetate, dioxane, acetonitrile, chloroform, dichloromethane, dimethylformamide, dimethyl sulfoxide, hexamethyl phosphoroamide, benzene, ether, water, and mixtures thereof. These materials are chosen according to the reaction.

The condensation reaction can be achieved by stirring for one hour to several days at -15 °C to room temperature, preferably for one hour to one day at 0 °C to room temperature.

As to the reaction to remove the protecting group, for example, reduction using Pd-C, it can be carried out for one hour to several days at -15 °C to room temperature, preferably for one hour to one day at 0 °C to room temperature. But when a strong acid such as hydrochloric acid is used, contact between the peptide product and the strong acid is preferably avoided; therefore the reaction is preferably carried out for from one to several hours.

## Examples

The following examples, which are illustrative only and not intended to limit the scope of the invention, describe the preparation of the compounds of the present invention. The NMR was measured using t-butanol ($\delta = 1.23$ ppm) as the internal standard and expressed in the $\delta$ value, and the specific rotatory power was measured using a sodium lamp ($\lambda = 5893$ Å).

### Example 1.

(i) To a suspension of 4.18 g of Z-Gly-OH and 5.12 g of DSC in 80 ml of acetonitrile, 1.58 g of pyridine in 20 ml of acetonitrile was added at room temperature and the mixture was stirred for 6 hrs to give Z-Gly-ONSu. Without purification, 7.31 g of TosOH.GABA-OBzl and 2.02 g of TEA were added to the solution at room temperature. After stirring for 20 hrs and evaporation in vacuo, the oily residue was dissolved in 100 ml of ethyl acetate. The solution was washed with water, 5% aqueous sodium hydrogencarbonate, brine, 10% aqueous citric acid solution, and brine. After drying over anhydrous sodium sulfate, the organic layer was evaporated in vacuo to dryness. The crystalline residue was recrystallized from ethyl acetate/ether to give 6.43 g of Z-Gly-GABA-OBzl; yield 84%.

m.p.: 83.5 - 85 °C

The above illustrates the method of preparation in the case of a known compound.

The following novel compounds were obtained in the same manner.

Z-Ala-GABA-OBzl

m.p.: 99.5 - 100.5 °C

$[\alpha]^{22} = +6.43$ (c = 1.0, DMF)

Z-Val-GABA-OBzl

m.p.: 114.5 - 116 °C

$[\alpha]^{25} = +10.1$ (c = 1.0, DMF)

Z-Leu-GABA-OBzl

m.p.: 73 - 74 °C

$[\alpha]^{25} = -3.8$ (c = 1.0, DMF)

Z-Ile-GABA-OBzl

m.p.: 106.5 - 109 °C

$[\alpha]^{25} = +6.7$ (c = 1.0, DMF)

(ii) 4.6 g of Z-Gly-GABA-OBzl was dissolved in a mixture of methanol (50 ml), acetic acid (30 ml) and water (5 ml), and hydrogenated in the presence of 2.0 g of 10% Pd-C at atmospheric pressure at room temperature. After stirring for 12 hrs and filtration, the reaction mixture was evaporated in vacuo to dryness. The residue was dissolved in a solution of ethanol (20 ml) and toluene (30 ml), and the remaining acetic acid was evaporated off together with the solvent in vacuo. The procedure was repeated three times. The crystalline residue was recrystallized from water/ethanol to give 2.05 g of Gly-GABA

m.p.: 212 - 215 °C (Decomposition)

NMR(0.1NDCl/$D_2O$): $\delta = 1.77$(tt,2H,$J_1 = 7.5$Hz,$J_2 = 6.8$Hz), 2.25(t,2H,J = 7.5Hz 3.25(t,2H,J = 6.8Hz), 3.77(s,1H)

The above illustrates the method of preparation in the case of a known compound.

The following novel compounds were obtained in the same manner.

Ala-GABA (Compound 1)

m.p.: 215 - 218 °C (Decomposition)

$[\alpha]^{22} = +15.1$ (c = 1.0, $H_2O$)

NMR(0.1NDCl/$D_2O$): $\delta = 1.50$(d,3H,J = 7.0Hz), 1.78(tt,2H,$J_1 = 7.0$Hz,$J_2 = 7.0$Hz), 2.29(t,2H,J = 7.0Hz), 3.24(dt,1H,$J_1 = 7.0$Hz,$J_2 = 14.0$Hz), 3.27(dt,1H,$J_1 = 7.0$Hz,$J_2 = 14.0$Hz), 4.02(q,1H,J = 7Hz)

Val-GABA (Compound 2)
m.p.: 202 - 203 °C
$[\alpha]^{22}$ = +40.7 (c=1.0, $H_2O$)
NMR(0.1NDCl/$D_2O$): δ=1.01(d,3H,J=7.0Hz), 1.02(d.3H,J=7.0Hz), 1.80(tt,2H,$J_1$=7.0Hz,$J_2$=7.0Hz), 2.12-2.24(m,1H), 2.31(t,2H,J=7.0Hz), 3.24(dt,1H,$J_1$=7.0Hz,$J_2$=14.0Hz), 3.30-(dt,1H,$J_1$=7.0Hz,$J_2$=14.0Hz), 3.72(d,1H,J=6.5Hz)
Leu-GABA (Compound 3)
m.p.: 179.5 - 180.5 °C
$[\alpha]^{24}$ = +31.6 (c=1.0, $H_2O$)
NMR(0.1NDCl/$D_2O$): δ=0.94(d,3H,J=6.0Hz), 0.96(d,3H,J=6.0Hz), 1.58-1.75(m,3H), 1.81-(tt,2H,$J_1$=7.0Hz,$J_2$=7.0Hz), 2.36(t,2H,J=7.0Hz), 3.22(dt,1H,$J_1$=7.0Hz,$J_2$=14.0Hz), 3.32-(dt,1H,$J_1$=7.0Hz,$J_2$=14.0Hz), 3.94(t,1H,J=7.5Hz)
Ile-GABA (Compound 4)
m.p.: 152 - 154 °C
$[\alpha]^{24}$ = +28.6 (c=1.0, $H_2O$)
NMR(0.1NDCl/$D_2O$): δ=0.92(t,3H), 0.98(d,3H), 1.17-1.29(m,1H), 1.45-1.56(m,1H), 1.80-(tt,2H,$J_1$=7.0Hz,$J_2$=7.0Hz), 1.89-2.00(m,1H), 2.34(t,2H,J=7.0Hz), 3.23(dt,1H,$J_1$=7.0Hz,$J_2$=14.0Hz),3.31-(dt,1H,$J_1$=7.0Hz,$J_2$=14.0Hz), 3.79(d,1H,J=6.0Hz)

Example 2.

(i) To a solution of 4.78 g of Z-Ser-OH and 4.34 g of HONB in 100 ml of THF, 4.54 g of DCC was added at 0 °C and the mixture was stirred at 0 °C for 1 h and at room temperature for 1 h to give Z-Ser-ONB. Precipitated DCUrea was filtered off and the filtrate was concentrated in vacuo. The oily product was dissolved in 100 ml of dioxane. 7.31 of TosOH.GABA-OBzl and 2.02 g of TEA were added to the solution at room temperature. After stirring for 20 hrs and evaporation in vacuo, the oily residue was dissolved in 100 ml of dichloromethane. The solution was washed with water, 5% sodium hydrogencarbonate aqueous solution, water, 10% citric acid aqueous solution, and water. After dehydration over sodium sulfate anhydride, the organic layer was evaporated in vacuo to dryness. The crystalline residue was recrystallized from ethyl acetate/ether to give 5.08 g of Z-Ser-GABA-OBzl; yield 61%.
m.p.: 137 - 138 °C
$[\alpha]^{25}$ = +17.5 (c=1.0, DMF)
The following compounds were obtained in the same manner.
Z-Thr-GABA-OBzl
m.p.: 94.5 - 95.5 °C
$[\alpha]^{25}$ = +2.0 (c=1.0, DMF)
Z-Tyr(Bzl)-GABA-OBzl
m.p.: 138 - 139 °C
$[\alpha]^{22}$ = -13.0 (c=1.0, DMF)
Z-Hyp-GABA-OBzl
m.p.: 73 - 75 °C
$[\alpha]^{22}$ = -15.0 (c=1.0, DMF)
Z-Phe-GABA-OBzl
m.p.: 133 - 134 °C
$[\alpha]^{22}$ = -8.9 (c=1.0, DMF)
Z-Phe-GABOB-OBzl
m.p.: 122 - 123 °C
$[\alpha]^{25}$ = -9.1 (c=1.0, DMF)
(ii) In the same manner as Example 1 (ii), 4.0 g of Z-Ser-GABA-OBzl was hydrogenated in the presence of Pd-C and the protecting group removed to give 1.50 g of Ser-GABA (Compound 5); yield 82%.
m.p.: 188 - 191 °C (Decomposition)
$[\alpha]^{24}$ = +12.5 (c=1.0, $H_2O$)
NMR(0.1NDCl/$D_2O$): δ=1.79(tt,2H,$J_1$=7.5Hz,$J_2$=7.5Hz), 2.29(t,2H,J=7.5Hz), 3.27(t,2H,J=7.5Hz), 3.91(dd,1H,$J_1$=6.0Hz,$J_2$=12.5Hz), 3.97(dd,1H,$J_1$=4.0Hz,$J_2$=12.5Hz), 4.07(dd,1H,$J_1$=4.0Hz,$J_2$=6.0Hz)
The following compounds were obtained in the same manner.
Thr-GABA (Compound 6)
m.p.: 136 °C (Decomposition)
$[\alpha]^{24}$ = +19.2 (c=1.0, $H_2O$)

NMR(0.1NDCl/$D_2O$): δ = 1.29(d,3H,J = 6.5Hz), 1.81(tt,2H,$J_1$ = 7.0Hz,$J_2$ = 7.0Hz), 2.37(t,2H,J = 7.0Hz), 3.28(t,2H,J = 7.0Hz), 3.79(d,1H,J = 6.0Hz), 4.12(dq,1H,$J_1$ = 6.0Hz,$J_2$ = 6.5Hz)

Tyr-GABA (Compound 7)

NMR(0.1NDCl/$D_2O$): δ = 1.48-1.61(m,2H), 1.97-2.13(m,2H), 2.97(dt,1H,$J_1$ = 7.0Hz,$J_2$ = 14.0Hz), 3.00-(dd,1H,$J_1$ = 9.0Hz,$J_2$ = 14.0Hz), 3.16(dd,1H,$J_1$ = 6.0Hz,$J_2$ = 14.0Hz), 3.29(dt,1H,$J_1$ = 7.0Hz,$J_2$ = 14.0Hz), 4.06(dd,1H,$J_1$ = 6.0Hz,$J_2$ = 9.0Hz), 6.86(d,2H,J = 8.0Hz), 7.13(d,2H,J = 8.0Hz)

Hyp-GABA (Compound 8)

m.p.: 210 - 211 °C

$[\alpha]^{24}$ = -35.8 (c = 1.0, $H_2O$)

NMR(0.1NDCl/$D_2O$): δ = 1.80(tt,2H,$J_1$ = 7.0Hz,$J_2$ = 7.0Hz), 2.15-(ddd,1H,$J_1$ = 3.5Hz,$J_2$ = 10.0Hz,$J_3$ = 14.0Hz),2.33(t,2H,J = 7.0Hz), 2.47-(dddd,1H,$J_1$ = 2.0Hz,$J_2$ = 2.0Hz,$J_3$ = 8.0Hz,$J_4$ = 14.0Hz), 3.25(dt,1H,$J_1$ = 7.0Hz,$J_2$ = 7.0Hz), 3.31-(dt,1H,$J_1$ = 7.0Hz,$J_2$ = 14.0Hz), 3.41(ddd,1H,$J_1$ = 2.0Hz,$J_2$ = 3.5Hz,$J_3$ = 12.5Hz), 4.53-(dd,1H,$J_1$ = 8.0Hz,$J_2$ = 10.0Hz), 4.68-4.72(m,1H)

Phe-GABA (Compound 9)

m.p.: 173.5 - 174.5 °C

$[\alpha]^{22}$ = +54.1 (c = 1.0, $H_2O$)

NMR(0.1NDCl/$D_2O$): δ = 1.46-1.63(m,2H), 1.94-2.11(m,2H), 2.98(dt,1H,$J_1$ = 7.0Hz,$J_2$ = 14.0Hz), 3.09-(dt,1H,$J_1$ = 9.0Hz,$J_2$ = 14.0Hz), 3.23(dd,1H,$J_1$ = 6.0Hz,$J_2$ = 13.0Hz), 3.25(dt,1H,$J_1$ = 7.0Hz,$J_2$ = 14.0Hz), 4.12(dd,1H,$J_1$ = 6.0Hz,$J_2$ = 9.0Hz), 7.24-7.42(m,5H)

Phe-GABOB (Compound 10)

m.p.: 177 °C (Decomposition)

$[\alpha]^{25}$ = +60.3 (c = 1.1, $H_2O$)

NMR(0.1NDCl/$D_2O$): δ = 2.09-2.32(m,2H), 3.10-3.29(m,4H), 3.88-4.35(m,1H), 4.17-4.23(m,1H), 7.25-7.45(m,5H)

Example 3.

(i) To a suspension of 6.47 g of Boc-Asp(OBzl)-OH and 5.64 g of DSC in 80 ml of acetonitrile, 1.74 g of pyridine in 20 ml of acetonitrile was added at room temperature and the mixture was stirred for 12 hrs to give Boc-Asp(OBzl)-ONSu. Without purification, 7.31 g of TosOH.GABA-OBzl and 2.02 g of TEA was added to the mixture at room temperature. After stirring for 20 hrs and evaporation in vacuo, the oily residue was dissolved in 100 ml of ethyl acetate. The solution was washed with water, 5% sodium hydrogencarbonate aqueous solution, brine, 10% citric acid aqueous solution, and brine. After dehydration over sodium sulfate anhydride, the organic layer was evaporated in vacuo to dryness. The crystalline residue was recrystallized from ethyl acetate/ether/petroleum ether to give 7.94 g of Boc-Asp(OBzl)-GABA-OBzl; yield 80%.

m.p.: 96 - 98 °C

$[\alpha]^{24}$ = -11.3 (c = 1.1, DMF)

(ii) 4.98 g of Boc-Asp(OBzl)-GABA-OBzl was dissolved in 50 ml of 98% formic acid and hydrogenated in the presence of 1.0 g of 10% of Pd-C at atmospheric pressure at room temperature. After stirring for 3 days and filtration, the reaction mixture was evaporated in vacuo to dryness. Toluene was added to the residue and the remaining formic acid was evaporated off together with the solvent. The procedure was repeated three times. The crystalline residue was recrystallized from water/ethanol to give 1.82 g of Asp-GABA (Compound 11); yield 83%.

m.p.: 216.5 - 218 °C (Decomposition)

$[\alpha]^{25}$ = +12.6 (c = 0.5, 0.1NNaOH)

NMR(0.1NDCl/$D_2O$): δ = 1.81(tt,2H,$J_1$ = 7.0Hz,$J_2$ = 7.0Hz), 2.40(t,2H,J = 7.0Hz), 2.95-(dd,1H,$J_1$ = 7.0Hz,$J_2$ = 18.0Hz),3.01(dd,1H,$J_1$ = 6.0Hz,$J_2$ = 18.0Hz), 3.23(dt,1H,$J_1$ = 7.0Hz,$J_2$ = 13.5Hz), 3.33-(dt,1H,$J_1$ = 7.0Hz,$J_2$ = 13.5Hz), 4.27(dd,1H,$J_1$ = 6.0Hz,$J_2$ = 7.0Hz)

Example 4.

(i) To a solution of 0.52 g of GABA and 0.51 g of TEA in water/acetonitrile (10 ml/10 ml), 1.73 g of Z-Pro-ONSu and 30 ml of acetonitrile were added at room temperature. After stirring for 20 hrs, the solvent was distilled in vacuo and the residue was dissolved in a mixture of 20 ml of 1N HCl aqueous solution and 50 ml of ethyl acetate. The organic layer was separated and washed with brine and extracted with 5% aqueous sodium hydrocarbonate. The extract was washed with ethyl acetate and acidified with 6N

aqueous HCl. The precipitated oil substance was extracted with ethyl acetate and the organic solution was washed with brine. After dehydration over anhydrous sodium sulfate and evaporation in vacuo, the oily residue was triturated with petroleum ether and the crystalline product was recrystallized from ethyl acetate/ether to give 0.89 g of Z-Pro-GABA; yield 53%.

m.p.: 73 - 75 °C

$[\alpha]^{22}$ = -29.5 (c = 1.0, DMF)

The following compounds were obtained in the same manner.

Z-Phe-EACA

m.p.: 126 - 128 °C

$[\alpha]^{25}$ = -10.2 (c = 1.0, DMF)

(ii) In the same manner as Example 1 (ii), 4.0 g of Z-Ser-GABA-OBzl was hydrogenated in the presence of Pd-C and the protecting group removed to give 1.50 g of Pro-GABA (Compound 12).

m.p.: 202 - 205 °C (Decomposition)

$[\alpha]^{24}$ = -47.3 (c = 1.0, $H_2O$)

NMR(0.1NDCl/$D_2O$): $\delta$ = 1.79(tt,2H,$J_1$ = 7.0Hz,$J_2$ = 7.0HZ), 1.97-2.11(m,3H), 2.29(t,2H,J = 7.0Hz), 2.36-2.49(m,1H), 3.24(dt,1H,$J_1$ = 7.0Hz,$J_2$ = 14.0HZ), 3.29(dt,1H,$J_1$ = 7.0Hz,$J_2$ = 14.0Hz), 3.34-3.77(m,2H), 4.32-(dd,1H,$J_1$ = 7.0Hz,$J_2$ = 8.0Hz)

The following compound was obtained in the same manner.

Phe-EACA (Compound 13)

m.p.: 130 °C (Decomposition)

$[\alpha]^{25}$ = +42.9 (c = 1.1, $H_2O$)

NMR(0.1NDCl/$D_2O$): $\delta$ = 0.97-1.07(m,2H), 1.22-1.34(m,2H), 1.48(tt,2H,$J_1$ = 7.0Hz,$J_2$ = 7.0Hz), 2.30-(t,2H,J = 7.0Hz), 2.91-3.00(m,1H), 3.15-3.23(m,1H), 3.08(dd,1H,$J_1$ = 9.0Hz,$J_2$ = 13.0Hz), 3.22-(dd,1H,$J_1$ = 6.0Hz,$J_2$ = 13.0Hz), 4.12(dd,1H,$J_1$ = 6.0Hz,$J_2$ = 9.0Hz), 7.20-7.45(m,5H)

Example 5.

(i) 2.02 g of TEA was added to a solution of 4.74 g of Boc-Pro-OH and 7.63 g of TosOH.GABOB-OBzl in 80 ml of dichloromethane. 4.54 g of DCC was added to the solution at 0 °C, and the solution was stirred for 3 hrs at 0 °C and overnight at room temperature. Precipitated DCUrea was filtered off and the filtrate was evaporated in vacuo. The residue was dissolved in ethyl acetate and washed with 10% citric acid aqueous solution, water, 4% sodium hydrogencarbonate aqueous solution, and brine. After dehydration over sodium sulfate, the organic layer was evaporated in vacuo to dryness to give 7.93 g of Boc-Pro-GABOB-OBzl; yield 98%.

m.p.: oily substance

$[\alpha]^{26}$ = -45.7 (c = 1.1, $CHCl_3$)

The following compounds were obtained in the same manner.

Boc-Pro-EACA-OBzl

m.p.: oily substance

$[\alpha]^{22}$ = -42.9 (c = 1.1, $CHCl_3$)

Boc-Phe-GABOB-OBzl

m.p.: 103 - 104 °C

$[\alpha]^{22}$ = +5.11 (c = 1.0, $CHCl_3$)

Boc-Phe-EACA-OBzl

m.p.: 102 - 103 °C

$[\alpha]^{22}$ = +4.25 (c = 1.0, $CHCl_3$)

(ii) To a solution of 2.50 g of Boc-Pro-GABOB-OBzl in 20 ml of dioxane, 20 ml of 6N HCl/dioxane was added at room temperature. After stirring for 4 hrs, the solvent was distilled in vacuo and the residue was dissolved in a mixture of methanol (40 ml), acetic acid (10 ml) and water (5 ml). 0.25 g of 10% Pd-C was added to the solution. After stirring overnight at atmospheric pressure at room temperature, the catalyst was filtered off and the filtrate was concentrated in vacuo. Water was added to the residue and insoluble matter was filtered off. After concentration of the filtrate, the oily residue was triturated with water/ethanol to give 1.08 g of Pro-GABOB (Compound 14); yield 81%.

m.p.: 210 - 212 °C

$[\alpha]^{26}$ = -40.5 (c = 1.3, 0.1NHCl)

NMR(0.1NDCl/$D_2O$) : $\delta$ = 2.01-2.11(m,3H), 2.41-2.54(m,2H), 2.58-2.65(m,1H), 3.30-3.48(m,3H), 4.13-4.22(m,1H), 4.37(dd,1H,$J_1$ = 7.0Hz,$J_2$ = 8.0Hz)

The following compounds were obtained in the same manner.

Pro-EACA (Compound 15)

m.p.: 182 - 183 °C

$[\alpha]^{25}$ = -47.6 (c = 1.0, $H_2O$)

NMR(0.1NDCl/$D_2O$): δ = 1.30-1.38(m,2H), 1.49-1.60(m,4H), 1.96-2.11(m,3H), 2.38(t,2H,J = 8.0Hz), 2.39-2.49(m,1H), 3.19(dt,1H,$J_1$ = 7.0Hz,$J_2$ = 14.0Hz), 3.29(dt,1H,$J_1$ = 7.0Hz,$J_2$ = 14.0Hz), 3.35-3.48(m,2H), 4.33(t,1H,J = 7.5Hz)

Phe-GABA (Compound 11)

Phe-EACA (Compound 13)

Example 6.

(i) To a suspension of 3.50 g of Boc-Gly-OH and 5.12 g of DSC in 80 ml of acetonitrile, 1.58 g of pyridine in 20 ml of acetonitrile was added at room temperature and the mixture was stirred for 4 hrs to give Boc-Gly-ONSu. Without purification, 7.31 g of TosOH.GABA-OBzl and 2.02 g of TEA were added to the mixture at room temperature. After stirring for 20 hrs and evaporation in vacuo, the oily residue was washed with water, 5% sodium hydrogencarbonate aqueous solution, brine, 10% citric acid aqueous solution, and brine. After dehydration over sodium sulfate anhydride, the organic layer was evaporated in vacuo. The oily residue was crystallized from petroleum ether and recrystallized from ethyl acetate/ether/petroleum ether to give 5.54 g of Boc-Gly-GABA-OBzl; yield 79%.

m.p.: 94.0 - 94.5 °C

(ii) 5.26 g of Boc-Gly-GABA-OBzl was treated with 75 ml of 4N HCl/dioxane at room temperature for 1.5 hrs. The solvent was distilled in vacuo and the oily residue was triturated with ether. The crystalline residue was collected by filtration and washed with ether. The product was dried over NaOH pellets in vacuo to give 4.21 g of HCl.Gly-GABA-OBzl. The resulting product was used without further purification.

(iii) 4.68 g of Z-Glu(ONSu)-OBzl was added to a solution of 3.15 g of HCl.Gly-GABA-OBzl and 1.11 g of TEA in 100 ml of acetonitrile at room temperature. After stirring for 20 hrs and evaporation in vacuo, the residue was dissolved in ethyl acetate. The solution was washed with water, 5% sodium hydrogencarbonate aqueous solution, brine, 10% citric acid aqueous solution, and brine. The solution was dried over sodium sulfate anhydride and evaporated in vacuo to dryness. The crystalline residue was recrystallized from ethyl acetate/ether to give 5.62 g of Z-Glu(Gly-GABA-OBzl)-OBzl.

m.p.: 107.5 - 109.5 °C

$[\alpha]^{24}$ = -7.5 (c = 1.0, DMF)

The following compounds were obtained in the same manner.

Z-Glu(Ala-GABA-OBzl)-OBzl

m.p.: 165.5 - 166.5 °C

$[\alpha]^{24}$ = -8.8 (c = 1.0, DMF)

Z-Glu(Ile-GABA-OBzl)-OBzl

m.p.: 166 - 170 °C

$[\alpha]^{24}$ = -5.3 (c = 1.1, DMF)

Z-Glu(Phe-GABA-OBzl)-OBzl

m.p.: 154 - 156 °C

$[\alpha]^{24}$ = -9.8 (c = 1.0, DMF)

Z-Glu(Tyr(OBzl)-GABA-OBzl)-OBzl

m.p.: 169 - 170.5 °C

$[\alpha]^{24}$ = -11.0 (c = 1.0, DMF)

Z-Glu(Pro-GABA-OBzl)-OBzl

m.p.: 99 - 100.5 °C

$[\alpha]^{24}$ = -36.0 (c = 1.0, DMF)

Z-Glu(Ser(OBzl)-GABA-OBzl)-OBzl

m.p.: 120 - 123 °C

$[\alpha]^{24}$ = -2.3 (c = 1.1, DMF)

Z-Glu(Asp(OBzl)-GABA-OBzl)-OBZl

m.p.: 129 - 131 °C

$[\alpha]^{24}$ = -17.3 (c = 1.0, DMF)

Z-Glu(Glu(OBzl)-GABA-OBzl)-OBzl

m.p.: 144 - 145.5 °C

$[\alpha]^{24}$ = -7.5 (c = 1.0, DMF)

Z-Glu(Lys(Z)-GABA-OBzl)-OBzl

m.p.: 140 °C (Decomposition)

$[\alpha]^{24}$ = -8.7 (c = 1.1, DMF)

(iv) 4.34 g of Z-Glu(Gly-GABA-OBzl)-OBzl was dissolved in a solution of methanol (40 ml), acetic acid (40 ml) and water (5 ml), and hydrogenated in the presence of 10% Pd-C (1.0 g) at atmospheric pressure at room temperature. After stirring for 2 days and filtration, the filtrate was evaporated in vacuo. 50 ml of toluene was added to the residue and the remaining acetic acid was coevaporated off with the solvent in vacuo. The procedure was repeated three times. The white precipitate was collected by filtration and recrystallized from water/ethanol to give 1.89 g of γ-Glu-Gly-GABA (Compound 16).

m.p.: 225 - 226 °C

$[\alpha]^{25}$ = +7.6 (c = 0.5, 0.1NNaOH)

NMR(0.1NDCl/$D_2O$): δ = 1.79(tt,2H,$J_1$ = 7.0Hz,$J_2$ = 7.0Hz), 2.16-2.31(m,2H), 2.39(t,2H,J = 7.0Hz), 2.55-(dt,1H,$J_1$ = 7.0Hz,$J_2$ = 16.0Hz), 2.60(dt,1H,$J_1$ = 7.0Hz,$J_2$ = 16.0Hz), 3.24(t,2H,J = 7.0Hz), 3.86(s,2H), 4.09-(t,1H,J = 6.5Hz)

The following compounds were obtained in the same manner.

γ-Glu-Ala-GABA (Compound 17)

m.p.: 187 - 188 °C

$[\alpha]^{22}$ = -26.5 (c = 1.0, $H_2O$)

NMR(0.1NDCl/$D_2O$): δ = 1.34(d,3H,J = 7.0Hz), 1.77(tt,2H,$J_1$ = 7.0Hz,$J_2$ = 7.0Hz), 2.10-2.25(m,2H), 2.37-(t,2H,J = 7.0Hz), 2.49(dt,1H,$J_1$ = 7.0Hz,$J_2$ = 15.0Hz),2.54(dt,1H,$J_1$ = 7.0Hz,$J_2$ = 15.0Hz), 3.20-(dt,1H,$J_1$ = 7.0Hz,$J_2$ = 14.0Hz), 3.24(dt,1H,$J_1$ = 7.0Hz,$J_2$ = 14.0Hz), 4.00(t,1H,J = 7.0Hz), 4.18-(q,1H,J = 7.0Hz)

γ-Glu-Ile-GABA (Compound 18)

m.p.: 187 - 190 °C (Decomposition)

$[\alpha]^{24}$ = -21.7 (c = 1.0, $H_2O$)

NMR(0.1NDCl/$D_2O$): δ = 0.86(t,3H,J = 7.5Hz), 0.90(d,3H,J = 7.0Hz), 1.13-1.22(m,1H), 1.41-1.52(m,1H), 1.75-1.86(m,3H), 2.12-2.26(m,2H), 2.39(t,2H,J = 7.0Hz), 2.52(dt,1H,$J_1$ = 8.0Hz,$J_2$ = 16.0Hz), 2.58-(dt,1H,$J_1$ = 8.0Hz,$J_2$ = 16.0Hz), 3.22(dt,1H,$J_1$ = 7.0Hz,$J_2$ = 14.0Hz), 3.26(dt,1H,$J_1$ = 7.0Hz,$J_2$ = 14.0Hz), 4.02-(t,1H,J = 6.5Hz), 4.05(d,1H,J = 8.0Hz)

γ-Glu-Phe-GABA.$H_2O$ (Compound 19)

m.p.: 174 - 175 °C (Decomposition)

$[\alpha]^{24}$ = +4.1 (c = 1.0, $H_2O$)

NMR(0.1NDCl/$D_2O$): δ = 1.50-1.62(m,2H), 2.00-2.18(m,4H), 2.48(t,2H,J = 7.5Hz), 2.97-3.05(m,1H), 3.02(d,2H,J = 8.0Hz), 3.20(dt,1H,$J_1$ = 7.0Hz,$J_2$ = 14.0Hz),3.98(t,1H,J = 6.5Hz), 4.47(t,1H,J = 8.0Hz), 7.24-7.38(m,5H)

γ-Glu-Pro-GABA (Compound 20)

m.p.: 55 °C (Decomposition)

$[\alpha]^{25}$ = -60.2 (c = 1.0, $H_2O$)

NMR(0.1NDCl/$D_2O$): δ = 1.79(tt,2H,$J_1$ = 7.0Hz,$J_2$ = 7.0Hz), 1.89-2.03(m,3H), 2.13-2.29(m,3H), 2.39-(t,H,J = 7.0Hz), 2.65(t,2H,J = 7.0Hz), 3.17-3.29(m,2H), 3.55-3.69(m,2H), 3.91(t,1H,J = 7.0Hz), 4.33-(dd,1H,$J_1$ = 7.0Hz,$J_2$ = 8.0Hz)

γ-Glu-Ser-GABA (Compound 21)

m.p.: 175 - 177 °C (Decomposition)

$[\alpha]^{22}$ = -15.6 (c = 1.0, $H_2O$)

NMR(0.1NDCl/$D_2O$): δ = 1.79(tt,2H,$J_1$ = 7.0Hz,$J_2$ = 7.0Hz), 2.20-(dddd,1H,$J_1$ = 7.0Hz,$J_2$ = 7.0Hz,$J_3$ = 7.0Hz,J4 = 14.0Hz), 2.26-(dddd,1H,$J_1$ = 7.0Hz,$J_2$ = 7.0Hz,$J_3$ = 7.0Hz,$J_4$ = 14.0Hz), 2.39(t,2H,J = 7.0Hz), 2.57-(ddd,1H,$J_1$ = 7.0Hz,$J_2$ = 7.0Hz,$J_3$ = 15.0Hz), 2.62(ddd,1H,$J_1$ = 7.0Hz,$J_2$ = 7.0Hz,$J_3$ = 15.0Hz), 3.23-(ddd,1H,$J_1$ = 7.0Hz,$J_2$ = 7.0Hz,$J_3$ = 13.0Hz), 3.28(ddd,1H,$J_1$ = 7.0Hz,$J_2$ = 7.0Hz,$J_3$ = 13.0Hz), 3.82-(d,1H,J = 5.5Hz), 4.08(t,1H,J = 7.0Hz), 4.35(t,1H,J = 5.5Hz)

γ-Glu-Tyr-GABA (Compound 22)

m.p.: 185 - 186 °C

$[\alpha]^{22}$ = +6.3 (c = 1.0, AcOH)

NMR(0.1NDCl/$D_2O$): δ = 1.50-1.61(m,2H), 2.01(2.26(m,4H), 2.49(t,1H,J = 8.0Hz), 2.50(t,1H,J = 8.0Hz), 2.91(dd,1H,$J_1$ = 8.0Hz,$J_2$ = 13.0Hz), 2.96(dd,1H,$J_1$ = 8.0Hz,$J_2$ = 13.0Hz), 2.95-3.03(m,1H), 3.21-(dt,1H,$J_1$ = 6.5Hz,$J_2$ = 14.0Hz), 4.01(t,1H,J = 6.5Hz), 4.40(t,1H,J = 8.0Hz), 6.82(d,2H,J = 8.0Hz), 7.12-(d,2H,J = 8.0Hz)

γ-Glu-Asp-GABA (Compound 23)

m.p.: 217 - 218 °C (Decomposition)

$[\alpha]^{22}$ = +1.0 (c = 0.5, 0.1NNaOH)

NMR(0.1NDCl/$D_2O$): δ = 1.78(tt,2H,$J_1$ = 7.0Hz,$J_2$ = 7.0HZ), 2.15-2.29(m,2H), 2.38(t,2H,J = 7.0Hz), 2.54-(dt,1H,$J_1$ = 7.5Hz,$J_2$ = 15.0Hz), 2.58(dt,1H,$J_1$ = 7.5Hz,$J_2$ = 15.0Hz), 2.81(dd,1H,$J_1$ = 8.0Hz,$J_2$ = 16.5Hz), 2.89(dd,1H,$J_1$ = 6.0Hz,$J_2$ = 16.5Hz), 3.25(t,2H,J = 7.0Hz), 4.09(t,1H,J = 7.0Hz), 4.64-(dd,1H,$J_1$ = 6.0Hz,$J_2$ = 8.0Hz)

γ-Glu-Glu-GABA (Compound 24)

m.p.: 179 - 183 °C (Decomposition)

$[\alpha]^{22}$ = -11.2 (c = 1.0, $H_2O$)

NMR(0.1NDCl/$D_2O$): δ = 1.79(tt,2H,$J_1$ = 7.0Hz,$J_2$ = 7.0Hz), 1.96-(ddt,1H,$J_1$ = 7.0Hz,$J_2$ = 9.0Hz,$J_3$ = 15.0Hz), 2.09(ddt,1H,$J_1$ = 6.0Hz,$J_2$ = 7.0Hz,$J_3$ = 15.0Hz), 2.14-2.25-(m,2H), 2.39(t,2H,J = 7.0Hz), 2.47(t,2H,J = 7.0Hz), 2.48-2.60(m,2H), 3.22(dt,1H,$J_1$ = 7.0Hz,$J_2$ = 14.0Hz), 3.25(dt,1H,$J_1$ = 7.0Hz,$J_2$ = 14.0Hz), 3.97(t,1H,J = 6.5Hz), 4.26(dd,1H,$J_1$ = 6.0Hz,$J_2$ = 9.0Hz)

γ-Glu-Lys-GABA (Compound 25)

m.p.: 277 - 278 °C (Decomposition)

$[\alpha]^{22}$ = -10.1 (c = 1.0, AcOH)

NMR(0.1NDCl/$D_2O$): δ = 1.33-1.50(m,2H), 1.63-1.79(m,4H), 1.79(tt,2H,$J_1$ = 7.0Hz,$J_2$ = 7.0Hz), 2.18-(dddd,1H,$J_1$ = 7.0Hz,$J_2$ = 7.0Hz,$J_3$ = 7.0Hz,$J_4$ = 15.0Hz), 2.22-(dddd,1H,$J_1$ = 7.0Hz,$J_2$ = 7.0Hz,$J_3$ = 7.0Hz,$J_4$ = 15.0Hz), 2.38(t,2H,J = 7.0Hz), 2.53-(ddd,1H,$J_1$ = 7.0Hz,$J_2$ = 7.0Hz,$J_3$ = 15.0Hz), 2.58(ddd,1H,$J_1$ = 7.0Hz,$J_2$ = 7.0Hz,$J_3$ = 15.0Hz), 2.98-(br.t,2H,J = 7.5Hz), 3.22(dt,1H,$J_1$ = 7.0Hz,$J_2$ = 13.0Hz),3.26(dt,1H,$J_1$ = 7.0Hz,$J_2$ = 13.0Hz), 4.04-(t,1H,J = 7.0Hz), 4.18(dd,1H,$J_1$ = 6.0Hz,$J_2$ = 8.0Hz)

Example 7.

(i) 1.62 g of Leu-GABA and 0.76 g of TEA were dissolved in a mixture of 30 ml of acetonitrile and 15 ml of water. 3.28 g of Z-Glu(ONSu)-OBzl and 20 ml of acetonitrile were added to the solution at room temperature with stirring. After stirring for 20 hrs and evaporation in vacuo, the residue was dissolved in a mixture of 50 ml of 1N HCl aqueous solution and 100 ml of chloroform. The organic layer was separated and washed with water and dehydrated over anhydrous sodium sulfate. The solvent was distilled in vacuo and crystalline residue was recrystallized from chloroform/n-hexane to give 3.88 g of Z-Glu(Leu-GABA-OH)-OBzl; yield 97%.

m.p.: 127.5 - 129 °C

$[\alpha]^{24}$ = -14.9 (c = 1.0, DMF)

The following compounds were obtained in the same manner.

Z-Glu(Val-GABA-OH)-OBzl

m.p.: 197 - 198 °C

$[\alpha]^{24}$ = -5.2 (c = 1.0, DMF)

Z-Glu(Hyp-GABA-OH)-OBzl

m.p.: 92 - 94 °C

$[\alpha]^{24}$ = -28.9 (c = 1.0, DMF)

(ii) In the same manner as Example 6 (iv), 4.34 g of Z-Glu(Leu-GABA-OH)-OBzl was hydrogenated in the presence of Pd-C and removed the protecting group to give 1.25 g of γ-Glu-Leu-GABA (Compound 26); yield 60%.

m.p.: 180 - 181 °C

$[\alpha]^{24}$ = -22.3 (c = 1.0, $H_2O$)

NMR(0.1NDCl/$D_2O$): δ = 0.87(d,3H,J = 6.0Hz), 0.92(d,3H,J = 6.0Hz), 1.50-1.67(m,3H), 1.78-(tt,2H,$J_1$ = 7.0Hz,$J_2$ = 7.0Hz), 2.13-2.27(m,2H), 2.38(t,2H,J = 7.0Hz), 2.52(dt,1H,$J_1$ = 7.5Hz,$J_2$ = 15.0Hz),2.58-(dt,1H,$J_1$ = 7.5Hz,$J_2$ = 15.0Hz), 3.20(dt,1H,$J_1$ = 7.0Hz,$J_2$ = 14.0Hz), 3.26(dt,1H,$J_1$ = 7.0Hz,$J_2$ = 14.0Hz), 4.06-(t,1H,J = 6.5Hz), 4.22(dd,1H,$J_1$ = 5.5Hz,$J_2$ = 9.5Hz)

The following compounds were obtained in the same manner.

γ-Glu-Val-GABA (Compound 27)

m.p.: 204 - 206 °C (Decomposition)

$[\alpha]^{25}$ = -23.8 (c = 1.0, $H_2O$)

NMR(0.1NDCl/$D_2O$): δ = 0.92(d,3H,J = 7.0Hz), 0.94(d,3H,J = 7.0Hz), 1.80(tt,2H,$J_1$ = 7.0Hz,$J_2$ = 7.0Hz), 2.04(dqq,1H,$J_1$ = 7.0Hz,$J_2$ = 7.0Hz,$J_3$ = 7.0Hz), 2.12-2.26(m,2H), 2.40(t,2H,J = 7.0Hz), 2.52-(dt,1H,$J_1$ = 7.5Hz,$J_2$ = 16.0Hz), 2.59(ddd,1H,$J_1$ = 7.0Hz,$J_2$ = 8.0Hz,$J_3$ = 16.0Hz), 3.22-(dt,1H,$J_1$ = 7.0Hz,$J_2$ = 13.5Hz), 3.99(d,1H,J = 7.0Hz), 4.00(t,1H,J = 7.0Hz)

γ-Glu-Hyp-GABA (Compound 28)

m.p.: 88 °C (Decomposition)
$[\alpha]^{25}$ = -50.3 (c = 1.0, $H_2O$)
NMR(0.1NDCl/$D_2O$): δ = 1.80(tt,2H,$J_1$ = 7.0Hz,$J_2$ = 7.0Hz), 2.02-2.09(m,1H), 2.13-2.36(m,1H), 2.41-(t,2H,J = 7.0Hz), 2.55-2.77(m,2H), 3.18-3.30(m,2H), 3.62(dd,1H,$J_1$ = 2.0Hz,$J_2$ = 11.0Hz), 3.79-(dd,1H,$J_1$ = 4.0Hz,$J_2$ = 11.0Hz), 4.09(t,1H,J = 7.5Hz), 4.43(dd,1H,$J_1$ = 8.0Hz,$J_2$ = 8.5Hz), 4.55-4.60(m,1H)

Example 8.

(i) To a mixture of 3.98 g of His.$H_2O$.HCl and 2.01 g of sodium carbonate in a mixture of 25 ml of DMF and 50 ml of $H_2O$, 7.27 g of Z-Glu(ONSu)-OBzl in 25 ml of acetonitrile was added at room temperature with stirring. After stirring for 20 hrs, the solvent was distilled and the residue was neutrallized with 20 ml of 1N HCl aqueous solution at 5 °C. The precipitated oil was extracted with 100 ml of chloroform and crystalline product was obtained as a precipitate. The resulting product was collected and washed with water and recrystallized from methanol aqueous solution to give 6.34 g of Z-Glu(His-OH)-OBzl; yield 80%.
m.p.: 134 - 136 °C
$[\alpha]^{24}$ = -7.2 (c = 1.0, DMF)
(ii) 2.47 g of DCC in 50 ml of dichloromethane was added to a mixture of 5.09 g of Z-Glu(His-OH)-OBzl, 4.02 g of TosOH.GABA-OBzl, 1.11 g of NMM and 2.15 g of HONB in 100 ml of dichloromethane at 0 °C. The mixture was stirred at 0 °C for 2 hrs and at room temperature for 20 hrs. Precipitated DCUrea and TosOH.NMM were filtered off and the filtrate was evaporated in vacuo. The oily residue was dissolved in 100 ml of chloroform, and the solution was washed with water, 5% sodium hydrogencarbonate aqueous solution, water, 10% citric acid aqueous solution and water. The solvent was distilled and the oily residue was crystallized from ether. The crude product was purified with silica gel column chromatography to give 4.87 g of Z-Glu(His-GABA-OBzl)-OBzl; yield 71%.
m.p.: 139 - 141 °C
$[\alpha]^{24}$ = -10.8 (c = 1.1, DMF)
(iii) In the same manner as Example 6 (iv), 4.1 g of Z-Glu(His-GABA-OBzl)-OBzl was hydrogenated in the presence of Pd-C and removed the protecting group to give 1.57 g of γ-Glu-His-GABA (Compound 29).
m.p.: 146 - 149 °C (Decomposition)
$[\alpha]^{25}$ = -3.0 (c = 1.0, $H_2O$)
NMR(0.1NDCl/$D_2O$): δ = 1.73(tt,2H,$J_1$ = 7.5Hz,$J_2$ = 7.5Hz), 2.14-2.27(m,2H), 2.32(t,2H,J = 7.5Hz), 2.48-(t,2H,J = 8.0Hz), 3.14(dd,1H,$J_1$ = 8.0Hz,$J_2$ = 15.0Hz),3.17-3.27(m,3H), 3.87(t,1H,J = 6.5Hz), 4.60-(dd,1H,$J_1$ = 7.0Hz,$J_2$ = 8.0Hz), 7.32(s,1H), 8.65(s,1H)

Example 9.

(i) 2.03 g of TEA was added to a mixture of 5.84 g of Boc-Phe-OH and 7.03 g of TosOH.β-Ala-OBzl in 80 ml of dichloroethane. 4.54 g of DCC was added to the solution at 0 °C. After stirring for 3 hrs at 0 °C and overnight at room temperature, precipitated DCUrea was filtered off and the solvent was distilled in vacuo. The residue was dissolved in ethyl acetate and the solution was washed with 10% citric acid aqueous solution, water, 5% sodium hydrogencarbonate aqueous solution and brine. After dehydration over sodium sulfate anhydride, the solvent was evaporated to give 8.36 g of Boc-Phe-β-Ala-OBzl; yield 98%.
(ii) 33 ml of 6N HCl/dioxane was added to a solution of 4.26 g of Boc-Phe-β-Ala-OBzl in 16 ml of dioxane. After stirring for 1 hr at room temperature, the solvent was distilled in vacuo and the residue was dissolved in chloroform. The solution was neutralized with an ice cooled saturated aqueous solution of sodium hydrogencarbonate aqueous solution. The water layer was extracted with chloroform. The procedure was repeated three times, and the chloroform layer was washed with brine. After dehydration over sodium sulfate anhydride, the solvent was distilled in vacuo. The residue was dissolved in 80 ml of dichloromethyl and 3.71 g of Z-Glu-OBzl was added to the solution. The solution was stirred for 3 hrs at 0 °C and overnight at room temperature. Precipitated DCUrea was filtered off and the filtrate was evaporated in vacuo to give 5.37 g of Z-Glu(Phe-β-Ala-OBzl)-OBzl; yield 77%.
m.p.: 161 - 162.5 °C
$[\alpha]^{22}$ = -12.0 (c = 1.0, $CHCl_3$)
The following compounds were obtained in the same manner.
Z-Glu(Phe-GABOB-OBzl)-OBzl
m.p.: 142.5 - 143.5 °C

[α]$^{22}$ = -7.2 (c = 1.0, $CHCl_3$)
Z-Glu(Phe-EACA-OBzl)-OBzl
m.p.: 145 - 146 °C
[α]$^{22}$ = -10.8 (c = 1.4, $CHCl_3$)
Z-Glu(Pro-β-Ala-OBzl)-OBzl
m.p.: 109 - 110.5 °C
[α]$^{22}$ = -57.2 (c = 1.2, $CHCl_3$)
Z-Glu(Pro-GABOB-OBzl)-OBzl
m.p.: oily substance
[α]$^{26}$ = -44.9 (c = 1.2, $CHCl_3$)
Z-Glu(Pro-EACA-OBzl)-OBzl
m.p.: oily substance
[α]$^{22}$ = -40.4 (c = 2.0, $CHCl_3$)
Z-Glu(OBzl)-Phe-β-Ala-OBzl
m.p.: 157 - 158 °C
[α]$^{22}$ = -20.1 (c = 1.0, $CHCl_3$)
Z-Glu(OBzl)-Pro-GABA-OBzl
m.p.: 142.5 - 143.5 °C
Z-Glu(OBzl)-Pro-EACA-OBzl
m.p.: oily substance
Z-Asp(Phe-β-Ala-OBzl)-OBzl
m.p.: 158 - 159 °C
[α]$^{22}$ = -2.2 (c = 1.0, $CHCl_3$)
Z-Asp(Phe-EACA-OBzl)-OBzl
m.p.: 133 - 133.5 °C
[α]$^{22}$ = +18.9 (c = 1.1, $CHCl_3$)
Z-Asp(OBzl)-Phe-β-Ala-OBzl
m.p.: 144 - 145 °C
[α]$^{22}$ = +10.7 (c = 1.0, $CHCl_3$)

(iii) In the same manner as Example 6 (iv), 2.50 g of Z-Glu(Phe-β-Ala-OBzl)-OBzl was hydrogenated in the presence of Pd-C and removed the protecting group to give 1.30 g of γ-Glu-Phe-β-Ala (Compound 30); yield 99%.

NMR(0.1NDCl/$D_2O$): δ = 2.07(dt,1H,$J_1$ = 7.0Hz,$J_2$ = 14.5Hz), 2.14(dt,1H,$J_1$ = 7.0Hz,$J_2$ = 14.5Hz), 2.36-(ddd,1H,$J_1$ = 5.5Hz,$J_2$ = 8.0Hz,$J_3$ = 17.5Hz), 2.45(ddd,1H,$J_1$ = 5.5Hz,$J_2$ = 6.0Hz,$J_3$ = 17.5Hz), 2.52-(t,2H,J = 7.0Hz), 2.99(dd,1H,$J_1$ = 8.0Hz,$J_2$ = 13.5Hz),3.03(dd,1H,$J_1$ = 8.0Hz,$J_2$ = 13.5Hz), 3.27-(ddd,1H,$J_1$ = 5.5Hz,$J_2$ = 8.0Hz,$J_3$ = 14.0Hz), 3.40(ddd,1H,$J_1$ = 5.5Hz,$J_2$ = 6.5Hz,$J_3$ = 14.0HZ), 3.97-(t,1H,J = 6.5Hz), 4.49(t,1H,J = 8.0Hz), 7.24(dt,2H,$J_1$ = 1.5Hz,$J_2$ = 7.0Hz), 7.30(tt,1H,$J_1$ = 1.5Hz,$J_2$ = 7.0Hz), 7.35(tt,2H,$J_1$ = 1.5Hz,$J_2$ = 7.0Hz)

The following compounds were obtained in the same manner.

γ-Glu-Phe-GABOB (Compound 31)
m.p.: 163 - 165 °C (Decomposition)
[α]$^{26}$ = +21.3 (c = 1.0, 0.1NHCl)
NMR(0.1NDCl/$D_2O$): δ = 2.09-2.19(m,2H), 2.19-2.27(m,1H), 2.31-2.33(m,1H), 2.30(br.t,2H,J = 6.5Hz), 3.00-3.14(m,2H), 3.14-3.35(m,2H), 3.91-4.03(m,2H), 4.51-4.55(m,1H), 7.20-7.39(m,5H)

γ-Glu-Phe-EACA (Compound 32)
m.p.: 184 - 185 °C (Decomposition)
[α]$^{26}$ = +14.1 (c = 1.1, 0.1NHCl)
NMR(0.1NDCl/$D_2O$): δ = 1.07(br.tt,2H,$J_1$ = 7.0Hz,$J_2$ = 7.0Hz), 1.27(br.tt,2H,$J_1$ = 7.0Hz,$J_2$ = 7.0Hz), 1.50-(br.tt,2H,$J_1$ = 7.0Hz,$J_2$ = 7.0Hz), 2.12(dt,2H,$J_1$ = 6.5Hz,$J_2$ = 7.5Hz), 2.33(t,2H,J = 7.5Hz), 2.49-(br.t,2H,J = 7.5Hz), 2.99(ddd,1H,$J_1$ = 6.5Hz,$J_2$ = 7.0Hz,$J_3$ = 13.5Hz), 3.01(d,2H,J = 8.0Hz), 3.14-(ddd,1H,$J_1$ = 6.5Hz,$J_2$ = 7.0Hz,$J_3$ = 13.5Hz), 4.00(t,1H,J = 6.5Hz), 4.48(t,1H,J = 8.0Hz), 7.26-(br.d,2H,J = 7.0Hz), 7.30(br.t,1H,J = 7.0Hz), 7.37(br.t,2H,J = 7.0Hz)

γ-Glu-Pro-β-Ala (Compound 33)
m.p.: 91 - 93 °C
[α]$^{26}$ = +50.1 (c = 1.1, 0.1NHCl)
NMR(0.1NDCl/$D_2O$) δ = 1.85-2.05(m,4H), 2.15-2.30(m,3H), 2.62(t,1H,J = 6.5Hz), 2.68(br.t,1H,J = 7.5Hz), 3.39-3.53(m,4H), 3.55-3.70(m,4H), 4.10(t,1H,J = 6.5Hz), 4.32(dd,1H,$J_1$ = 4.5Hz,$J_2$ = 8.0Hz)

γ-Glu-Pro-GABOB (Compound 34)

m.p.: 66 - 68 °C
[α]$^{26}$ = -32.0 (c = 1.2, 0.1NHCl)
NMR(0.1NDCl/$D_2O$): δ = 1.90-2.04(m,3H), 2.15-2.33(m,3H), 2.42-2.51(m,1H), 2.57-2.64(m,1H), 2.69-(br.t,2H,J = 7.0Hz), 3.31-3.35(m,2H), 3.59-3.68(m,2H), 4.08-4.20(m,2H), 4.15(dd,1H,$J_1$ = 5.0Hz,$J_2$ = 9.0Hz)

γ-Glu-Pro-EACA (Compound 35)
m.p.: 62 - 63 °C
[α]$^{26}$ = -40.5 (c = 1.1, 0.1NHCl)
NMR(0.1NDCl/$D_2O$) δ = 1.27-1.36(m,2H), 1.46-1.65(m,4H), 1.86-2.03(m,3H), 2.15-2.30(m,3H), 2.38-(t,2H,J = 7.5Hz), 2.68(dt,2H,$J_1$ = 3.0Hz,$J_2$ = 8.0Hz), 3.11-3.26(m,2H), 3.58-3.68(m,2H), 4.13(t,1H,J = 7.0Hz), 4.32(dd,1H,$J_1$ = 5.0Hz,$J_2$ = 8.5Hz)

Glu-Phe-β-Ala (Compound 36)
m.p.: 154 - 155.5 °C
[α]$^{26}$ = +35.6 (c = 0.9, 0.1NHCl)
NMR(0.1NDCl/$D_2O$): δ = 2.15(dt,2H,$J_1$ = 7.5Hz,$J_2$ = 8.0Hz), 2.28-(ddd,1H,$J_1$ = 5.5Hz,$J_2$ = 8.0Hz,$J_3$ = 17.0Hz), 2.41(ddd,1H,$J_1$ = 5.5Hz,$J_2$ = 6.5Hz,$J_3$ = 17.0Hz), 2.51-(t,2H,J = 7.5Hz), 2.99(dd,1H,$J_1$ = 9.0Hz,$J_2$ = 13.0Hz),3.12(dd,1H,$J_1$ = 7.0Hz,$J_2$ = 13.0Hz), 3.20-(ddd,1H,$J_1$ = 5.5Hz,$J_2$ = 8.0Hz,$J_3$ = 13.5Hz), 3.39(ddd,1H,$J_1$ = 5.5Hz,$J_2$ = 6.5Hz,$J_3$ = 13.5Hz), 4.07-(t,1H,J = 6.5Hz), 4.52(dd,1H,$J_1$ = 7.0Hz,$J_2$ = 9.0Hz), 7.26(br.t,2H,J = 7.2Hz), 7.30(br.t,1H,J = 7.2Hz), 7.37-(br.t,2H,J = 7.2Hz)

Glu-Pro-GABA (Compound 47)
m.p.: 157 - 158 °C
[α]$^{26}$ = -60.1 (c = 1.0, $H_2O$)

Glu-Pro-EACA (Compound 48)
m.p.: 76 - 78 °C (Decomposition)
[α]$^{26}$ = -62.8 (c = 1.0, 0.1NHCl)
NMR(0.1NDCl/$D_2O$): δ = 1.23-1.33(m,2H), 1.42-1.65(m,4H), 1.79-2.07(m,3H), 2.17-2.31(m,2H), 2.14-(dd,1H,$J_1$ = 7.0Hz,$J_2$ = 14.5Hz), 2.34(t,2H,J = 7.5Hz), 2.58(t,2H,J = 7.5Hz), 3.10-(dd,1H,$J_1$ = 6.5Hz,$J_2$ = 14.0Hz),3.24(dd,1H,$J_1$ = 7.0Hz,$J_2$ = 14.0Hz), 3.55-3.65(m,1H), 3.65-3.75(m,1H), 4.36-(dd,1H,$J_1$ = 7.0Hz,$J_2$ = 8.0Hz), 4.41(dd,1H,$J_1$ = 5.0Hz,$J_2$ = 7.0Hz)

β-Asp-Phe-β-Ala (Compound 39)
m.p.: 158 - 160 °C (Decomposition)
[α]$^{26}$ = +13.4 (c = 1.2, 0.1NHCl)
NMR(0.1NDCl/$D_2O$): δ = 2.35(ddd,1H,$J_1$ = 5.5Hz,$J_2$ = 8.0Hz,$J_3$ = 17.0Hz), 2.44-(ddd,1H,$J_1$ = 5.5Hz,$J_2$ = 6.0Hz,$J_3$ = 17.0Hz), 3.00(dd,1H,$J_1$ = 5.5Hz,$J_2$ = 9.5Hz), 3.02(d,2H,J = 8.0Hz), 3.04-(dd,1H,$J_1$ = 5.5Hz,$J_2$ = 9.5Hz), 3.27(ddd,1H,$J_1$ = 5.5Hz,$J_2$ = 8.0Hz,$J_3$ = 14.0Hz), 3.40-(ddd,1H,$J_1$ = 5.5Hz,$J_2$ = 6.0Hz,$J_3$ = 14.0Hz), 4.33(t,1H,J = 5.5Hz), 4.54(t,1H,J = 8.0Hz), 7.23-(dt,2H,$J_1$ = 1.5Hz,$J_2$ = 6.5Hz), 7.32(tt,1H,$J_1$ = 1.5Hz,$J_2$ = 6.5Hz), 7.36(tt,2H,$J_1$ = 1.5Hz,$J_2$ = 6.5Hz)

β-Asp-Phe-EACA (Compound 40)
m.p.: 178 - 180 °C (Decomposition)
[α]$^{26}$ = +10.0 (c = 1.1, 0.1NHCl)
NMR(0.1NDCl/$D_2O$): δ = 0.97-1.10(m,2H), 1.20-1.35(m,2H), 1.49(tt,2H,$J_1$ = 7.5Hz,$J_2$ = 7.5Hz), 2.32-(br.t,2H,J = 7.5Hz), 2.94(dd,1H,$J_1$ = 13.0Hz,$J_2$ = 14.0Hz), 2.99(dd,1H,$J_1$ = 8.0Hz,$J_2$ = 18.0Hz), 3.00-(dd,1H,$J_1$ = 9.0Hz,$J_2$ = 13.5Hz), 3.07(dd,1H,$J_1$ = 5.5Hz,$J_2$ = 18.0Hz), 3.10(dd,1H,$J_1$ = 7.0Hz,$J_2$ = 13.5Hz), 3.12(dd,1H,$J_1$ = 13.0Hz,$J_2$ = 14.0Hz), 4.34(dd,1H,$J_1$ = 5.5Hz,$J_2$ = 8.0Hz), 4.52(dd,1H,$J_1$ = 7.0Hz,$J_2$ = 9.0Hz), 7.27(br,t,2H,J = 7.0Hz), 7.31(br,t,1H,J = 7.0Hz), 7.37(br,t,2H,J = 7.0Hz)

Asp-Phe-EACA (Compound 41)
m.p.: 168 - 171 °C (Decomposition)
[α]$^{26}$ = +14.9 (c = 1.0, 0.1NHCl)
NMR(0.1NDCl/$D_2O$): δ = 2.31(ddd,1H,$J_1$ = 5.5Hz,$J_2$ = 8.0Hz,$J_3$ = 17.0Hz), 2.41-(td,1H,$J_1$ = 5.5Hz,$J_2$ = 17.0Hz), 2.99(dd,1H,$J_1$ = 7.2Hz,$J_2$ = 18.0Hz), 3.01(dd,1H,$J_1$ = 8.0Hz,$J_2$ = 16.0Hz), 3.07(dd,1H,$J_1$ = 5.0Hz,$J_2$ = 18.0Hz), 3.10(dd,1H,$J_1$ = 7.0Hz,$J_2$ = 16.0Hz), 2.22-(ddd,1H,$J_1$ = 5.5Hz,$J_2$ = 8.0Hz,$J_3$ = 14.0Hz), 3.39(dt,1H,$J_1$ = 5.5Hz,$J_2$ = 14.0Hz), 4.33-(dd,1H,$J_1$ = 5.0Hz,$J_2$ = 7.2Hz), 4.53(br.t,1H,J = 8.0Hz), 7.25(d,2H,J = 7.0Hz), 7.31(t,1H,J = 7.0Hz), 7.37-(t,2H,J = 7.0Hz)

Example 10.

(i) To an ice cooled solution of 10.76 g of Boc-Pro-OH and 5.06 g of TEA in 250 ml of THF, 5.43 g of ECF was added dropwise at -15 °C. The mixture was stirred at the temperature for 15 min. A solution of 20.1 g of TosOH.GABA-OBzl and 5.57 g of TEA in 100 ml of chloroform was added to the mixture at 0 °C. The mixture was stirred at 0 °C for 2 hrs and at room temperature for 20 hrs. The solvent was evaporated and residue was dissolved in 150 ml of ethyl acetate and the solution was washed with water, 5% sodium hydrogencarbonate aqueous solution, brine, 10% citric acid aqueous solution and brine. The organic layer was dried over sodium sulfate anhydride and evaporated in vacuo to give oily Boc-Pro-GABA-OBzl.

The resulting Boc-Pro-GABA-OBzl was treated with 250 ml of 4N HCl/dioxane at room temperature for 2 hrs. The solvent and HCl were distilled in vacuo. The residue was dissolved in 100 ml of dioxane and remaining HCl was coevaporated off with the solvent. The oily residue was crystallized from ethanol/petroleum ether to give 15.48 g of HCl.Pro-GABA-OBzl; yield 95%.

(ii) Using HCl.Pro-GABA-OBzl and Z-Glu(ONSu)-OBzl, the condensation and reduction were carried out in the same manner as Example 6 (iii) and (iv) to give γ-Glu-Pro-GABA (Compound 23).

Example 11.

(i) To an ice cooled solution of 8.93 g of Z-Leu-OH.DCHA and 8.66 g of TosOH.EACA-OBzl in 100 ml of dichloromethane, 4.22.g of WSCD.HCl was added with stirring. After stirring for 2 hrs at 0 °C and 20 hrs at room temperature, precipitated DCHA.TosOH was filtered off and the filtrate was evaporated in vacuo. The oily residue was dissolved in 100 ml of ethyl acetate and the solution was washed with water, 5% sodium hydrogencarbonate aqueous solution, brine, 10% citric acid aqueous solution, and brine. After dehydration over anhydrous sodium sulfate. the solvent was distilled in vacuo. The oily residue was crystallized from petroleum ether to give 7.51 g of Z-Leu-EACA-OBzl; yield 80%.

Z-Leu-EACA-OBzl
m.p.: 88 - 89 °C
$[\alpha]^{25}$ = -5.1 (c = 1.0, DMF)

The following compounds were obtained in the same manner.

Z-Ile-EACA-OH
m.p.: 124 - 125 °C
$[\alpha]^{25}$ = +5.7 (c = 1.0, DMF)

Z-Pyr-EACA-OH
m.p.: 105 - 106 °C
$[\alpha]^{25}$ = +7.3 (c = 1.0, DMF)

Z-Gly-β-Ala-OBzl
m.p.: 81 - 82 °C

Z-Ala-β-Ala-OBzl
m.p.: 92 - 93 °C
$[\alpha]^{25}$ = +6.8 (c = 1.0, DMF)

Z-Val-β-Ala-OBzl
m.p.: 116 - 117 °C
$[\alpha]^{25}$ = +9.4 (c = 1.0, DMF)

Z-Leu-β-Ala-OBzl
m.p.: 75 - 76 °C
$[\alpha]^{25}$ = -3.2 (c = 1.0, DMF)

Z-Ser-β-Ala-OBzl
m.p.: 101.5 - 102.5 °C
$[\alpha]^{25}$ = +15.5 (c = 1.0, DMF)

Z-Thr-β-Ala-OBzl
m.p.: 85 - 87 °C
$[\alpha]^{25}$ = +5.9 (c = 1.0, DMF)

Z-Hyp-β-Ala-OBzl
m.p.: 99 - 100 °C
$[\alpha]^{25}$ = -18.0 (c = 1.0, DMF)

Z-Asp(OBzl)-β-Ala-OBzl
m.p.: 91 - 92 °C

$[\alpha]^{25}$ = -5.6 (C = 1.0, DMF)
Z-Pyr-$\beta$-Ala-OBzl
m.p.: 91 - 92 °C
$[\alpha]^{25}$ = +2.1 (c = 1.0, DMF)
Z-Lys(Z)-GABA-OBzl
m.p.; 116 - 118 °C
$[\alpha]^{25}$ = -3.1 (c = 1.0, DMF)
Z-Asp(GABA-OBzl)-OBzl
m.p.: 105 - 107 °C
$[\alpha]^{25}$ = -8.5 (c = 1.0, DMF)
Boc-D-Asp(OBzl)-GABA-OBzl
m.p.: 81 - 82 °C
$[\alpha]^{25}$ = +10.9 (c = 1.0, DMF)

(ii) In the same manner as Example 1 (ii), 6.0 g of Z-Leu-GABA-OBzl was hydrogenated in the presence of Pd-C and removed the protecting group to give 2.37 g of Leu-EACA.$^{1}/_{2}H_2O$ (Compound 42); yield 73%.
m.p.: 146 - 148 °C
$[\alpha]^{25}$ = +17.0 (c = 1.0, $H_2O$)
The following compounds were obtained in the same manner.
Ile-EACA (Compound 43)
m.p.: 160 - 162 °C
$[\alpha]^{25}$ = +17.8 (c = 1.0, $H_2O$)
Pyr-EACA (Compound 44)
m.p.: 130 - 131 °C
$[\alpha]^{25}$ = -23.2 (c = 1.0, $H_2O$)
$\beta$-Asp-GABA (Compound 45)
m.p.: 205 - 207 °C (Decomposition)
$[\alpha]^{25}$ = -14.7 (c = 0.5, 0.1NNaOH)
In the same manner as Method 1 (3), D-Asp-GABA (Compound 46 was obtained.
m.p.: 212 - 213.5 °C (Decomposition)
$[\alpha]^{25}$ = -12.5 (c = 0.5, 0.1NNaOH)

Example 12.

(i) HCl.Phe-GABA-OBzl was obtained in the same manner as Example 9 (i) and (ii). 2.11 g of WSCD.HCl was added to the mixture of 3.57 g of Z-Asp(OBzl)-OH, 4.15 g of HCl.Phe-GABA-OBzl and 1.11 g of TEA in an ice cooled water. The reaction mixture was stirred at 0 °C for 2 hrs and at room temperature for 20 hrs, and washed with water, 10% citric acid aqueous solution, water, 5% sodium hydrogencarbonate aqueous solution and brine. After dehydration over anhydrous sodium sulfate, the solvent was distilled in vacuo and crystal was collected from ether to give 5.31 g of Z-Asp(OBzl)-Phe-GABA-OBzl; yield 78%.
m.p.: 120 - 130 °C
$[\alpha]^{25}$ = -30.2 (c = 1.0, DMF)
The following compounds were obtained in the same manner.
Z-Asp(Phe-GABA-OBzl)-OBzl
m.p.: 163 - 165 °C
$[\alpha]^{25}$ = -55.4 (c = 1.0, DMF)
Z-Glu(OBzl)-Phe-GABA-OBzl
m.p.: 129 - 131 °C
$[\alpha]^{25}$ = -16.7 (c = 1.0, DMF)
Boc-D-Asp(OBzl)-Phe-GABA-OBzl
m.p.: 96 - 97 °C
$[\alpha]^{25}$ = -10.9 (c = 1.0, DMF)

(ii) In the same manner as Example 6 (iv), 3.74 g of Z-Asp(OBzl)-Phe-GABA-OBzl was hydrogenated in the presence of Pd-C and the protecting group removed to give 1.39 g of Asp-Phe-GABA (Compound 47); yield 69%.
m.p.: 217 - 218 °C (Decomposition)
$[\alpha]^{25}$ = +16.0 (c = 1.0, $H_2O$)
$\beta$-Asp-Phe-GABA (Compound 48)

m.p.: 216 - 217 °C (Decomposition)
$[\alpha]^{25}$ = -22.1 (c = 0.5, 0.1NNaOH)

$\beta$-Asp-Phe-GABA (Compound 49)
m.p.: 196 - 198 °C (Decomposition)
$[\alpha]^{25}$ = -15.2 (c = 0.5, 0.05NNaOH)

In the same manner as Method I(3), D-Asp-Phe-GABA (Compound 50 was obtained.
m.p.: 187 - 189 °C (Decomposition)
$[\alpha]^{25}$ = -19.3 (c = 0.5, 0.1NNaOH)

pharmaceutical studies of the compounds of the present invention were carried out as follows:

(I) The neurotropic activity of the peptide compounds of the present invention was examined by the method of Maria-S. Oitzl and J. P. Huston [Brain research, 308, 33-42 (1984)].

Wistar-strain rats were implanted with a set of electrodes for recording EEG under anesthesia with pentobarbital (50 mg/kg, i.p.). The electrodes were bilaterally implanted into the hippocampus (Bregma -2.7 mm, Lateral 2.5 mm, Depth 2.8 mm) by reference to THE RAT BRAIN IN STEREOTAXIC COODINATES (George Paxion & Charles Watson, Academic press). Chemitorode, a injection guide cannula connected with a recording electrode, was implanted in the temporoparietal left hole. The test drugs were unilaterally injected into the hippocampus of freely moving rats to examine their effect on EEG activity.

The rats were kept for about a week for a cure, and then used for the experiments. After the EEG activity was recorded for dozens of minutes in a normal state, 100 ng to 10 µg of the test drug dissolved in 2 µl of saline was injected into the hippocampus through the injection guide cannula. Only saline was used as a control. Immediately after administration recordinq of the EEG activity was commenced.

EEG peaks were observed even when the rat was in a still state. About 8 min after administration of the compounds of the present invention, characteristic spikes appeared and continued until 40 to 60 min after administration. In the case of GABA which is well-known as a depressive neurotransmitter having cerebral metabolic and sedative activity, a few minutes after administration similar EEG peaks appeared and continued until about 25 min after administration.

During the appearance of the peaks the behavior of the animal was observed. As a result, a sedated state was observed. However, it was not cataleptiform akinesia but natural and physiologic sedative state which immediately resulted in wakefulness on external stimulation.

As is shown by the results of the above pharmacological tests, the compounds of the present invention showed excellent neurotropic effect even at doses of the compounds on the order of one hundredth to one tenth of the dose of GABA. Furthermore, the effect of the compounds of the present invention continued longer than that of GABA.

(2) Analgesic effect

Groups of 10 ddY-strain male mice weighing 24-30 g were used for measurement of analgesic effect of the peptide compounds of the present invention by modified Randall-Selitto's test (Tale pressure method). Analgesic test was performed at 5 min after intracisternal injection of the test drugs. A result was shown in Table 1.

Table 1

| Drug | Dose (μmole/mouse) | Pain Index |
|---|---|---|
| Control | - | 1.17 ± 0.12 |
| Compound 1 | 0.1 | 1.80 ± 0.15 |
| Compound 9 | 0.1 | 1.89 ± 0.14 |
| Compound 19 | 0.1 | 1.72 ± 0.15 |
| GABA | 0.1 | 1.65 ± 0.13 |

$$\text{Pain Index} = \frac{\text{Pain threshold (g) after the administration}}{\text{Pain threshold (g) before the administration}}$$

The compounds of the present invention have sedative activity and show EEG peaks similar to those of GABA. The compounds of this invention have a GABA-like neurotropic effect, they are therefore useful as pharmaceutical agents having for example sedative, cerebral metabolic or analgesic activity or the like. Furthermore, the dipeptide compounds of the present invention are also useful as starting materials or intermediates for the preparation the tripeptide compounds of this invention.

## Claims

**Claims for the following Contracting States : AT, BE, CH, DE, FR, GB, IT, LI, LU, NL, SE**

**1.** A pharmaceutical composition containing as an active ingredient a peptide having the formula (I)

A-X-NH-Y-CO-R

where:
A is hydrogen, an amino protecting group, an acidic amino acid residue, or an acidic amino acid residue having a protecting group, and wherein:
(i) when A is hydrogen or an amino protecting group:
X is selected from Ala, Val, Leu, Ile, Pro, Ser, Thr, Tyr, Hyp, Asp, D-Asp, $\beta$-Asp, D-$\beta$-Asp, Phe and Pyr, with or without a protecting group;
Y is straight $C_{3-6}$ alkylene or hydroxyalkylene, and
R is a hydroxy group or a carboxy-protecting group;
provided that Pyr-GABA is excluded;
(ii) when A is an acidic amino acid residue or an acidic amino acid having a protecting group:
X is an amino acid residue with or without a protecting group;
Y is straight $C_{2-6}$ alkylene or hydroxyalkylene, and
R is a hydroxy group or a carboxy-protecting group;
or a pharmaceutically acceptable salt or complex thereof.

**2.** A composition according to claim 1(i) wherein Y is a straight alkylene group having 3 carbon atoms.

**3.** A composition according to claim 1(i) wherein Y is a straight alkylene group having 5 carbon atoms.

**4.** A composition according to claim 1(ii) wherein Y is a straight alkylene group having 2 carbon atoms.

**5.** A composition according to claim 1(ii) wherein Y is a straight alkylene group having 3 carbon atoms.

6. A composition according to claim 1(ii) wherein Y is a straight alkylene group having 5 carbon atoms.

7. A composition according to claim 1(ii) or any of claims 4 to 6 wherein X is a phenylalanine residue with or without a protecting group.

8. A composition according to claim 1(ii) or any of claims 4 to 6 wherein X is a proline residue with or without a protecting group.

9. A composition according to any preceding claim wherein X is a phenylalanine residue with or without a protecting group.

10. A composition according to claim 1 containing as an active ingredient a peptide selected from:
Ala-GABA, Val-GABA, Leu-GABA, Ile-GABA, Phe-GABA, Pro-GABA, Ser-GABA, Thr-GABA, Tyr-GABA, Hyp-GABA, Asp-GABA, β-Asp-GABA, D-Asp-GABA, D-β-Asp-GABA,
Ala-GABOB, Val-GABOB, Leu-GABOB, Ile-GABOB, Phe-GABOB, Pro-GABOB, Ser-GABOB, Thr-GABOB, Tyr-GABOB, Hyp-GABOB, Asp-GABOB, β-Asp-GABOB, D-Asp-GABOB, D-β-Asp-GABOB, Pyr-GABOB,
Ala-EACA, Val-EACA, Leu-EACA, Ile-EACA, Phe-EACA, Pro-EACA, Ser-EACA, Thr-EACA, Hyp-EACA, Asp-EACA, β-Asp-EACA, D-Asp-EACA, D-β-Asp-EACA, Pyr-EACA,
γ-Glu-Gly-GABA, γ-Glu-Ala-GABA, γ-Glu-Val-GABA,
γ-Glu-Leu-GABA, γ-Glu-Ile-GABA, γ-Glu-Phe-GABA,
γ-Glu-Pro-GABA, γ-Glu-Ser-GABA, γ-Glu-Thr-GABA,
γ-Glu-Tyr-GABA, γ-Glu-Hyp-GABA, γ-Glu-Lys-GABA,
γ-Glu-His-GABA, γ-Glu-Arg-GABA, γ-Glu-Asp-GABA,
γ-Glu-Glu-GABA, γ-Glu-β-Asp-GABA,
γ-Glu-y-Glu-GABA, γ-Glu-D-Asp-GABA,
γ-Glu-D-β-Asp-GABA, γ-Glu-Trp-GABA, γ-Glu-5-HTP-GABA, γ-Glu-Cys-GABA, γ-Glu-Met-GABA,
γ-Glu-Phe-β-Ala, γ-Glu-Phe-GABOB, γ-Glu-Phe-EACA,
Glu-Phe-GABA, Glu-Phe-β-Ala, Glu-Phe-GABOB, Glu-Phe-EACA, Asp-Phe-GABA, Asp-Phe-β-Ala, Asp-Phe-GABOB, Asp-Phe-EACA, β-Asp-Phe-GABA, β-Asp-Phe-β-Ala, β-Asp-Phe-GABOB, β-Asp-Phe-EACA, D-Asp-Phe-GABA, D-Asp-Phe-β-Ala, D-Asp-Phe-GABOB, D-Asp-Phe-EACA, D-β-Asp-Phe-GABA, D-β-Asp-Phe-β-Ala, D-β-Asp-Phe-GABOB, D-β-Asp-Phe-EACA,
γ-Glu-Pro-β-ALa, γ-Glu-Pro-GABOB, γ-Glu-Pro-EACA,
Glu-Pro-GABA, Glu-Pro-β-Ala, Glu-Pro-GABOB, Glu-Pro-EACA, Asp-Pro-GABA, Asp-Pro-β-Ala, Asp-Pro-GABOB, Asp-Pro-EACA, β-Asp-Pro-GABA, β-Asp-Pro-β-Ala, β-Asp-Pro-GABOB, β-Asp-Pro-EACA, D-Asp-Pro-GABA, D-Asp-Pro-β-Ala, D-Asp-Pro-GABOB, D-Asp-Pro-EACA, D-β-Asp-Pro-GABA, D-β-Asp-Pro-β-Ala, D-β-Asp-Pro-GABOB, D-β-Asp-Pro-EACA,
or a pharmaceutically acceptable salt or complex thereof.

11. A composition according to any preceding claim having neurotropic activity.

12. A neurotropic composition according to claim 11 having sedative activity.

13. A neurotropic composition according to claim 11 having cerebral metabolic activity.

14. A neurotropic composition according to claim 11 having analgesic activity.

15. A neurotropic composition according to claim 11 comprising Ala-GABA, Phe-GABA, or y-Glu-Phe-GABA.

16. The use of a peptide of formula (I) as specified in any of claims 1 to 9 or a pharmaceutically acceptable salt or complex thereof for the manufacture of agents having sedative, cerebral metabolic, or analgesic activity.

17. A peptide having the formula (I)

A-X-NH-Y-CO-R

where:

A is hydrogen, an amino protecting group, an acidic amino acid residue, or an acidic aminoacid residue having a protecting group, and wherein:

(i) when A is hydrogen or an amino protecting group:
X is selected from Ala, Val, Leu, Ile, Pro, Ser, Thr, Tyr, Hyp, Asp, D-Asp, β-Asp, D-β-Asp, and Pyr, with or without a protecting group;
Y is straight $C_{3-6}$ alkylene or hydroxyalkylene, and
R is a hydroxy group or a carboxy-protecting group;
provided that Pyr-GABA and Tyr-GABOB are excluded;
(ii) when A is an acidic amino acid residue or an acidic amino acid having a protecting group:
X is an amino acid residue with or without a protecting group;
Y is straight $C_{2-6}$ alkylene or hydroxyalkylene, and
R is a hydroxy group or a carboxy-protecting group;
or a pharmaceutically acceptable salt or complex thereof.

**18.** A peptide, salt or complex according to claim 17(i)wherein Y is a straight alkylene group having 3 carbon atoms.

**19.** A peptide, salt or complex according to claim 17(i) wherein Y is a straight alkylene group having 5 carbon atoms.

**20.** A peptide, salt or complex according to claim 17(ii) wherein Y is a straight alkylene group having 2 carbon atoms.

**21.** A peptide, salt or complex according to claim 17(ii) wherein Y is a straight alkylene group having 3 carbon atoms.

**22.** A peptide, salt or complex according to claim 17(ii) wherein Y is a straight alkylene group having 5 carbon atoms.

**23.** A peptide, salt or complex according to claim 17(ii) or any of claims 20 to 22 wherein X is a phenylalanine residue with or without a protecting group.

**24.** A peptide, salt or complex according to claim 17(ii) or any of claims 20 to 22 wherein X is a proline residue with or without a protecting group.

**25.** Ala-GABA, Val-GABA, Leu-GABA, Ile-GABA, Pro-GABA, Ser-GABA, Thr-GABA, Tyr-GABA, Hyp-GABA, Asp-GABA, β-Asp-GABA, D-Asp-GABA, D-β-Asp-GABA,
Ala-GABOB, Val-GABOB, Leu-GABOB, Ile-GABOB, Pro-GABOB, Ser-GABOB, Thr-GABOB, Tyr-GABOB, Hyp-GABOB, Asp-GABOB, β-Asp-GABOB, D-Asp-GABOB, D-β-Asp-GABOB, Pyr-GABOB,
Ala-EACA, Val-EACA, Leu-EACA, Ile-EACA, Pro-EACA, Ser-EACA, Thr-EACA, Hyp-EACA, Asp-EACA, β-Asp-EACA, D-Asp-EACA,
D-β-Asp-EACA, Pyr-EACA,
γ-Glu-Gly-GABA, γ-Glu-Ala-GABA, γ-Glu-Val-GABA,
γ-Glu-Leu-GABA, γ-Glu-Ile-GABA, γ-Glu-Phe-GABA,
γ-Glu-Pro-GABA, γ-Glu-Ser-GABA, γ-Glu-Thr-GABA,
γ-Glu-Tyr-GABA, γ-Glu-Hyp-GABA, γ-Glu-Lys-GABA,
γ-Glu-His-GABA, γ-Glu-Arg-GABA, γ-Glu-Asp-GABA,
γ-Glu-Glu-GABA, γ-Glu-β-Asp-GABA,
γ-Glu- -Glu-GABA, γ-Glu-D-Asp-GABA,
γ-Glu-D-β-Asp-GABA, γ-Glu-Trp-GABA, γ-Glu-5-HTP-GABA, γ-Glu-Cys-GABA, γ-Glu-Met-GABA,
γ-Glu-Phe-β-Ala, γ-Glu-Phe-GABOB, γ-Glu-Phe-EACA,
Glu-Phe-GABA, Glu-Phe-β-Ala, Glu-Phe-GABOB, Glu-Phe-EACA, Asp-Phe-GABA, Asp-Phe-β-Ala, Asp-Phe-GABOB, Asp-Phe-EACA, β-Asp-Phe-GABA, β-Asp-Phe-β-Ala, β-Asp-Phe-GABOB, β-Asp-Phe-EACA, D-Asp-Phe-GABA, D-Asp-Phe-β-Ala, D-Asp-Phe-GABOB, D-Asp-Phe-EACA, D-β-Asp-Phe-GABA, D-β-Asp-Phe-β-Ala, D-β-Asp-Phe-GABOB, D-β-Asp-Phe-EACA,
γ-Glu-Pro-β-Ala, γ-Glu-Pro-GABOB, γ-Glu-Pro-EACA,
Glu-Pro-GABA, Glu-Pro-β-Ala, Glu-Pro-GABOB, Glu-Pro-EACA, Asp-Pro-GABA, Asp-Pro-β-Ala, Asp-Pro-GABOB, Asp-Pro-EACA, β-Asp-Pro-GABA, β-Asp-Pro-β-Ala, β-Asp-Pro-GABOB, β-Asp-Pro-EACA, D-Asp-Pro-GABA, D-Asp-Pro-β-Ala, D-Asp-Pro-GABOB, D-Asp-Pro-EACA, D-β-Asp-Pro-GABA, D-β-Asp-Pro-β-Ala, D-β-Asp-Pro-GABOB, D-β-Asp-Pro-EACA,

**26.** A process for the preparation of a peptide according to claim 17 comprising the steps of coupling a partial peptide or peptides or an amino acid or amino acids with the corresponding remaining moiety so as to constitute the compound of formula (I) and if necessary removing any protecting group.

**Claims for the following Contracting States : ES, GR**

**1.** A process for the preparation of a peptide having the formula (I)

A-X-NH-Y-CO-R

where:

A is hydrogen, an amino protecting group, an acidic amino acid residue, or an acidic aminoacid residue having a protecting group, and wherein:

(i) when A is hydrogen or an amino protecting group:

X is selected from Ala, Val, Leu, Ile, Pro, Ser, Thr, Tyr, Hyp, Asp, D-Asp, β-Asp, D-β-Asp, and Pyr, with or without a protecting group;

Y is straight $C_{3-6}$ alkylene or hydroxyalkylene, and

R is a hydroxy group or a carboxy-protecting group;

provided that Pyr-GABA and Tyr-GABOB are excluded;

(ii) when A is an acid amino acid residue or an acidic amino acid having a protecting group:

X is an amino acid residue with or without a protecting group;

Y is straight $C_{2-6}$ alkylene or hydroxyalkylene, and

R is a hydroxy group or a carboxy-protecting group;

or a pharmaceutically acceptable salt or complex thereof,

comprising the steps of coupling a partial peptide or peptides or an amino acid or amino acids with the corresponding remaining moiety so as to constitute the compound of formula (I) and if necessary removing any protecting group.

**2.** A process according to claim 1(i)wherein Y is a straight alkylene group having 3 carbon atoms.

**3.** A process according to claim 1(i) wherein Y is a straight alkylene group having 5 carbon atoms.

**4.** A process according to claim 1(ii) wherein Y is a straight alkylene group having 2 carbon atoms.

**5.** A process according to claim 1(ii) wherein Y is a straight alkylene group having 3 carbon atoms.

**6.** A process according to claim 1(ii) wherein Y is a straight alkylene group having 5 carbon atoms.

**7.** A process according to claim 1(ii) or any of claims 2 to 4 wherein X is a phenylalanine residue with or without a protecting group.

**8.** A process according to claim 1(ii) or any of claims 2 to 4 wherein X is a proline residue with or without a protecting group.

**9.** A process according to claim 1 wherein the peptide is:

Ala-GABA, Val-GABA, Leu-GABA, Ile-GABA, Pro-GABA, Ser-GABA, Thr-GABA, Tyr-GABA, Hyp-GABA, Asp-GABA, β-Asp-GABA, D-Asp-GABA, D-β-Asp-GABA,

Ala-GABOB, Val-GABOB, Leu-GABOB, Ile-GABOB, Pro-GABOB, Ser-GABOB, Thr-GABOB, Tyr-GABOB, Hyp-GABOB, Asp-GABOB, β-Asp-GABOB, D-Asp-GABOB, D-β-Asp-GABOB, Pyr-GABOB,

Ala-EACA, Val-EACA, Leu-EACA, Ile-EACA, Pro-EACA, Ser-EACA, Thr-EACA, Hyp-EACA, Asp-EACA, β-Asp-EACA, D-Asp-EACA,

D-β-Asp-EACA, Pyr-EACA,

γ-Glu-Gly-GABA, γ-Glu-Ala-GABA, γ-Glu-Val-GABA,

γ-Glu-Leu-GABA, γ-Glu-Ile-GABA, γ-Glu-Phe-GABA,

γ-Glu-Pro-GABA, γ-Glu-Ser-GABA, γ-Glu-Thr-GABA, γ-Glu-Tyr-GABA, γ-Glu-Hyp-GABA, γ-Glu-Lys-GABA, γ-Glu-His-GABA, γ-Glu-Arg-GABA, γ-Glu-Asp-GABA, γ-Glu-Glu-GABA, γ-Glu-β-Asp-GABA, γ-Glu-γ-Glu-GABA, γ-Glu-D-Asp-GABA, γ-Glu-D-β-Asp-GABA, γ-Glu-Trp-GABA, γ-Glu-5-HTP-GABA, γ-Glu-Cys-GABA, γ-Glu-Met-GABA, γ-Glu-Phe-β-Ala, γ-Glu-Phe-GABOB, γ-Glu-Phe-EACA, Glu-Phe-GABA, Glu-Phe-β-Ala, Glu-Phe-GABOB, Glu-Phe-EACA, Asp-Phe-GABA, Asp-Phe-β-Ala, Asp-Phe-GABOB, Asp-Phe-EACA, β-Asp-Phe-GABA, β-Asp-Phe-β-Ala, β-Asp-Phe-GABOB, β-Asp-Phe-EACA, D-Asp-phe-GABA, D-Asp-Phe-β-Ala, D-Asp-Phe-GABOB, D-Asp-Phe-EACA, D-β-Asp-Phe-GABA, D-β-Asp-Phe-β-Ala, D-β-Asp-Phe-GABOB, D-β-Asp-Phe-EACA, γ-Glu-Pro-β-Ala, γ-Glu-Pro-GABOB, γ-Glu-Pro-EACA, Glu-Pro-GABA, Glu-Pro-β-Ala, Glu-Pro-GABOB, Glu-Pro-EACA, Asp-Pro-GABA, Asp-Pro-β-Ala, Asp-Pro-GABOB, Asp-Pro-EACA, β-Asp-Pro-GABA, β-Asp-Pro-β-Ala, β-Asp-Pro-GABOB, β-Asp-Pro-EACA, D-Asp-Pro-GABA, D-Asp-Pro-β-Ala, D-Asp-Pro-GABOB, D-Asp-Pro-EACA, D-β-Asp-Pro-GABA, D-β-Asp-Pro-β-Ala, D-β-Asp-Pro-GABOB, D-β-Asp-Pro-EACA,
or a pharmaceutically acceptable salt or complex thereof.

**Patentansprüche**

**Patentansprüche für folgende Vertragsstaaten : AT, BE, CH, DE, FR, GB, IT, LI, LU, NL, SE**

**1.** Pharmazeutische Zusammensetzung, die als Wirkstoff ein Peptid mit der Formel (I) enthält

A-X-NH-Y-CO-R

wo:

A Wasserstoff ist, eine Aminoschutzgruppe, ein saurer Aminosäurerest oder ein saurer Aminosäurerest mit einer Schutzgruppe, und worin:

(i) wenn A Wasserstoff oder eine Aminoschutzgruppe ist:
X aus Ala, Val, Leu, Ile Pro, Ser, Thr, Tyr, Hyp, Asp, D-Asp, β-Asp, D-β-Asp, Phe und Pyr, mit oder ohne Schutzgruppe ausgewählt wird;
Y gerades oder $C_{3-6}$-Alkylen oder Hydroxyalkylen und R eine Hydroxygruppe oder Carboxyschutzgruppe ist;
unter der Bedingung, dass Pyr-GABA ausgenommen ist;

(ii) wenn A ein saurer Aminosäurerest oder eine saure Aminosäure mit Schutzgruppe ist:
X ein Aminosäurerest mit oder ohne Schutzgruppe ist;
Y gerades $C_{2-6}$-Alkylen oder Hydroxyalkylen, und R eine Hydroxygruppe oder eine Carboxyschutzgruppe ist;

oder ein pharmazeutisch annehmbares Salz oder Komplex davon.

**2.** Zusammensetzung nach Anspruch 1(i), worin Y eine gerade Alkylengruppe mit 3 Kohlenstoffatomen ist.

**3.** Zusammensetzung nach Anspruch 1(i), worin Y eine gerade Alkylengruppe mit 5 Kohlenstoffatomen ist.

**4.** Zusammensetzung nach Anspruch 1(ii), worin Y eine gerade Alkylengruppe mit 2 Kohlenstoffatomen ist.

**5.** Zusammensetzung nach Anspruch 1(ii), worin Y eine gerade Alkylengruppe mit 3 Kohlenstoffatomen ist.

**6.** Zusammensetzung nach Anspruch 1(ii), worin Y eine gerade Alkylengruppe mit 5 Kohlenstoffatomen ist.

**7.** Zusammensetzung nach Anspruch 1(ii) oder einem der Ansprüche 4 bis 6, worin X ein Phenylalaninrest mit oder ohne eine Schutzgruppe ist.

**8.** Zusammensetzung nach Anspruch 1(ii) oder einem der Ansprüche 4 bis 6, worin X ein Prolinrest mit oder ohne eine Schutzgruppe ist.

9. Zusammensetzung nach einem der vorangehenden Ansprüche, worin X ein Phenylalaninrest mit oder ohne eine Schutzgruppe ist.

10. Zusammensetzung nach Anspruch 1, die als Wirkstoff ein Peptid enthält, das aus den folgenden ausgewählt ist:
Ala-GABA, Val-GABA, Leu-GABA, Ile-GABA, Phe-GABA, Pro-GABA, Ser-GABA, Thr-GABA, Tyr-GABA, Hyp-GABA, Asp-GABA, β-Asp-GABA, D-Asp-GABA, D-β-Asp-GABA,
Ala-GABOB, Val-GABOB, Leu-GABOB, Ile-GABOB, Phe-GABOB, Pro-GABOB, Ser-GABOB, Thr-GABOB, Tyr-GABOB, Hyp-GABOB, Asp-GABOB, β-Asp-GABOB, D-Asp-GABOB, D-β-Asp-GABOB, Pyr-GABOB, Ala-EACA, Val-EACA, Leu-EACA, Ile-EACA, Phe-EACA, Pro-EACA, Ser-EACA, Thr-EACA, Hyp-EACA, Asp-EACA, β-Asp-EACA, D-Asp-EACA, D-β-Asp-EACA, Pyr-EACA,
γ-Glu-Gly-GABA, γ-Glu-Ala-GABA, γ-Glu-Val-GABA,
γ-Glu-Leu-GABA, γ-Glu-Ile-GABA, γ-Glu-Phe-GABA,
γ-Glu-Pro-GABA, γ-Glu-Ser-GABA, γ-Glu-Thr-GABA,
γ-Glu-Tyr-GABA, γ-Glu-Hyp-GABA, γ-Glu-Lys-GABA,
γ-Glu-His-GABA, γ-Glu-Arg-GABA, γ-Glu-Asp-GABA,
γ-Glu-Glu-GABA, γ-Glu-β-Asp-GABA,
γ-Glu-γ-Glu-GABA, γ-Glu-D-Asp-GABA,
γ-Glu-D-β-Asp-GABA, γ-Glu-Trp-GABA, γ-Glu-5-HTP-GABA, γ-Glu-Cys-GABA, γ-Glu-Met-GABA,
γ-Glu-Phe-β-Ala, γ-Glu-Phe-GABOB, γ-Glu-Phe-EACA, Glu-Phe-GABA, Glu-Phe-β-Ala, Glu-Phe-GABOB, Glu-Phe-EACA, Asp-Phe-GABA, Asp-Phe-β-Ala, Asp-Phe-GABOB, Asp-Phe-EACA, β-Asp-Phe-GABA, β-Asp-Phe-β-Ala, β-Asp-Phe-GABOB, β-Asp-Phe-EACA,
D-Asp-Phe-GABA, D-Asp-Phe-β-Ala, D-Asp-Phe-GABOB, D-Asp-Phe-EACA,
D-β-Asp-Phe-GABA, D-β-Asp-Phe-β-Ala, D-β-Asp-Phe-GABOB, D-β-Asp-Phe-EACA,
γ-Glu-Pro-β-Ala, γ-Glu-Pro-GABOB, γ-Glu-Pro-EACA, Glu-Pro-GABA, Glu-Pro-β-Ala, Glu-Pro-GABOB, Glu-Pro-EACA, Asp-Pro-GABA, Asp-Pro-β-Ala, Asp-Pro-GABOB, Asp-Pro-EACA, β-Asp-Pro-GABA, β-Asp-Pro-β-Ala, β-Asp-Pro-GABOB, β-Asp-Pro-EACA,
D-Asp-Pro-GABA, D-Asp-Pro-β-Ala, D-Asp-Pro-GABOB, D-Asp-Pro-EACA,
D-β-Asp-Pro-GABA, D-β-Asp-Pro-β-Ala, D-β-Asp-Pro-GABOB, D-β-Asp-Pro-EACA,
oder ein pharmazeutisch annehmbares Salz oder Komplex davon.

11. Zusammensetzung nach einem der vorangehenden Ansprüche mit neurotropischer Aktivität.

12. Neurotropische Zusammensetzung nach Anspruch 11 mit beruhigender Aktivität.

13. Neurotropische Zusammensetzung nach Anspruch 11 mit Hirnstoffwechselaktivität.

14. Neurotropische Zusammensetzung nach Anspruch 11 mit schmerzstillender Wirkung.

15. Neurotropische Zusammensetzung nach Anspruch 11, die Ala-GABA, Phe-GABA, oder γ-Glu-Phe-GABA umfasst.

16. Verwendung eines Peptids der Formel (I), wie in einem der Ansprüche 1 bis 9 aufgeführt, oder eines pharmazeutisch annehmbaren Salzes oder Komplexes davon, zur Herstellung eines Agens mit beruhigender, Hirnstoffwechsel- oder schmerzstillender Aktivität.

17. Peptid mit der Formel (I)

A-X-NH-Y-CO-R

wo:
A Wasserstoff ist, eine Aminoschutzgruppe, ein saurer Aminosäurerest oder ein saurer Aminosäurerest mit einer Schutzgruppe, und worin:
(i) wenn A Wasserstoff oder eine Aminoschutzgruppe ist:
X aus Ala, Val, Leu, Ile Pro, Ser, Thr, Tyr, Hyp, Asp, D-Asp, β-Asp, D-β-Asp, Phe und Pyr, mit oder ohne Schutzgruppe ausgewählt wird;
Y gerades oder $C_{3-6}$-Alkylen oder Hydroxyalkylen und R eine Hydroxygruppe oder Carboxyschutzgruppe ist;

unter der Bedingung, dass Pyr-GABA und Tyr-GABOB ausgenommen sind;
(ii) wenn A ein saurer Aminosäurerest oder eine Aminosäure mit Schutzgruppe ist:
X ein Aminosäurerest mit oder ohne Schutzgruppe ist;
Y gerades $C_{2-6}$-Alkylen oder Hydroxyalkylen, und R eine Hydroxygruppe oder eine Carboxyschutzgruppe ist;
oder ein pharmazeutisch annehmbares Salz oder Komplex davon.

**18.** Peptid, Salz oder Komplex nach Anspruch 17(i), worin Y eine gerade Alkylengruppe mit 3 Kohlenstoffatomen ist.

**19.** Peptid, Salz oder Komplex nach Anspruch 17(i), worin Y eine gerade Alkylengruppe mit 5 Kohlenstoffatomen ist.

**20.** Peptid, Salz oder Komplex nach Anspruch 17(ii), worin Y eine gerade Alkylengruppe mit 2 Kohlenstoffatomen ist.

**21.** Peptid, Salz oder Komplex nach Anspruch 17(ii), worin Y eine gerade Alkylengruppe mit 3 Kohlenstoffatomen ist.

**22.** Peptid, Salz oder Komplex nach Anspruch 17(ii), worin Y eine gerade Alkylengruppe mit 5 Kohlenstoffatomen ist.

**23.** Peptid, Salz oder Komplex nach Anspruch 17(ii) oder einem der Ansprüche 20 bis 22, worin X ein Phenylalaninrest mit oder ohne eine Schutzgruppe ist.

**24.** Peptid, Salz oder Komplex nach Anspruch 17(ii) oder einem der Ansprüche 20 bis 22, worin X ein Prolinrest mit oder ohne eine Schutzgruppe ist.

**25.** Ala-GABA, Val-GABA, Leu-GABA, Ile-GABA, Pro-GABA, Ser-GABA, Thr-GABA, Tyr-GABA, Hyp-GABA, Asp-GABA, β-Asp-GABA, D-Asp-GABA, D-β-Asp-GABA,
Ala-GABOB, Val-GABOB, Leu-GABOB, Ile-GABOB, Pro-GABOB, Ser-GABOB, Thr-GABOB, Tyr-GABOB, Hyp-GABOB, Asp-GABOB, β-Asp-GABOB, D-Asp-GABOB, D-β-Asp-GABOB, Pyr-GABOB, Ala-EACA, Val-EACA, Leu-EACA, Ile-EACA, Pro-EACA, Ser-EACA, Thr-EACA, Hyp-EACA, Asp-EACA, β-Asp-EACA, D-Asp-EACA, D-β-Asp-EACA, Pyr-EACA,
γ-Glu-Gly-GABA, γ-Glu-Ala-GABA, γ-Glu-Val-GABA,
γ-Glu-Leu-GABA, γ-Glu-Ile-GABA, γ-Glu-Phe-GABA,
γ-Glu-Pro-GABA, γ-Glu-Ser-GABA, γ-Glu-Thr-GABA,
γ-Glu-Tyr-GABA, γ-Glu-Hyp-GABA, γ-Glu-Lys-GABA,
γ-Glu-His-GABA, γ-Glu-Arg-GABA, γ-Glu-Asp-GABA,
γ-Glu-Glu-GABA, γ-Glu-β-Asp-GABA,
γ-Glu-γ-Glu-GABA, γ-Glu-D-Asp-GABA,
γ-Glu-D-β-Asp-GABA, γ-Glu-Trp-GABA, γ-Glu-5-HTP-GABA, γ-Glu-Cys-GABA, γ-Glu-Met-GABA,
γ-Glu-Phe-β-Ala, γ-Glu-Phe-GABOB, γ-Glu-Phe-EACA, Glu-Phe-GABA, Glu-Phe-β-Ala, Glu-Phe-GABOB, Glu-Phe-EACA, Asp-Phe-GABA, Asp-Phe-β-Ala, Asp-Phe-GABOB, Asp-Phe-EACA, β-Asp-Phe-GABA, β-Asp-Phe-β-Ala, β-Asp-Phe-GABOB, β-Asp-Phe-EACA,
D-Asp-Phe-GABA, D-Asp-Phe-β-Ala, D-Asp-Phe-GABOB, D-Asp-Phe-EACA,
D-β-Asp-Phe-GABA, D-β-Asp-Phe-β-Ala, D-β-Asp-Phe-GABOB, D-β-Asp-Phe-EACA,
γ-Glu-Pro-β-Ala, γ-Glu-Pro-GABOB, γ-Glu-Pro-EACA, Glu-Pro-GABA, Glu-Pro-β-Ala, Glu-Pro-GABOB, Glu-Pro-EACA, Asp-Pro-GABA, Asp-Pro-β-Ala, Asp-Pro-GABOB, Asp-Pro-EACA, β-Asp-Pro-GABA, β-Asp-Pro-β-Ala, β-Asp-Pro-GABOB, β-Asp-Pro-EACA,
D-Asp-Pro-GABA, D-Asp-Pro-β-Ala, D-Asp-Pro-GABOB, D-Asp-Pro-EACA,
D-β-Asp-Pro-GABA, D-β-Asp-Pro-β-Ala, D-β-Asp-Pro-GABOB, D-β-Asp-Pro-EACA.

**26.** Verfahren zur Zubereitung eines Peptids nach Anspruch 17, das die Schritte umfasst, ein Teilpeptid oder Teilpeptide oder eine Aminosäure oder Aminosäuren mit dem entsprechenden übrigen Teil zu verbinden, so dass die Verbindung der Formel (I) gebildet wird und falls nötig alle Schutzgruppen zu entfernen.

**Patentansprüche für folgende Vertragsstaaten : ES, GR**

1. Verfahren zur Zubereitung eines Peptids mit der Formel (I)

A-X-NH-Y-CO-R

wo:

A Wasserstoff ist, eine Aminoschutzgruppe, ein saurer Aminosäurerest oder ein saurer Aminosäurerest mit einer Schutzgruppe, und worin:

(i) wenn A Wasserstoff oder eine Aminoschutzgruppe ist:

X aus Ala, Val, Leu, Ile Pro, Ser, Thr, Tyr, Hyp, Asp, D-Asp, β-Asp, D-β-Asp, und Pyr, mit oder ohne Schutzgruppe ausgewählt wird;

Y gerades oder $C_{3-6}$-Alkylen oder Hydroxyalkylen und R eine Hydroxygruppe oder Carboxyschutzgruppe ist;

unter der Bedingung, dass Pyr-GABA und Tyr-GABOB ausgenommen sind;

(ii) wenn A ein saurer Aminosäurerest oder eine Aminosäure mit Schutzgruppe ist:

X ein Aminosäurerest mit oder ohne Schutzgruppe ist;

Y gerades $C_{2-6}$-Alkylen oder Hydroxyalkylen, und R eine Hydroxygruppe oder eine Carboxyschutzgruppe ist;

oder ein pharmazeutisch annehmbares Salz oder Komplex davon,

das die Schritte umfasst, ein Teilpeptid oder Teilpeptide oder eine Aminosäure oder Aminosäuren mit dem entsprechenden übrigen Teil zu verbinden, so dass die Verbindung der Formel (I) gebildet wird und falls nötig alle Schutzgruppen zu entfernen.

2. Verfahren nach Anspruch 1(i), worin Y eine gerade Alkylengruppe mit 3 Kohlenstoffatomen ist.

3. Verfahren nach Anspruch 1(i), worin Y eine gerade Alkylengruppe mit 5 Kohlenstoffatomen ist.

4. Verfahren nach Anspruch 1(ii), worin Y eine gerade Alkylengruppe mit 2 Kohlenstoffatomen ist.

5. Verfahren nach Anspruch 1(ii), worin Y eine gerade Alkylengruppe mit 3 Kohlenstoffatomen ist.

6. Verfahren nach Anspruch 1(ii), worin Y eine gerade Alkylengruppe mit 5 Kohlenstoffatomen ist.

7. Verfahren nach Anspruch 1(ii) oder einem der Ansprüche 2 bis 4, worin X ein Phenylalaninrest mit oder ohne eine Schutzgruppe ist.

8. Zusammensetzung nach Anspruch 1(ii) oder einem der Ansprüche 2 bis 4, worin X ein Prolinrest mit oder ohne eine Schutzgruppe ist.

9. Verfahren nach Anspruch 1 worin das Peptid eines der folgenden ist:
Ala-GABA, Val-GABA, Leu-GABA, Ile-GABA, Pro-GABA, Ser-GABA, Thr-GABA, Tyr-GABA, Hyp-GABA, Asp-GABA, β-Asp-GABA, D-Asp-GABA, D-β-Asp-GABA,
Ala-GABOB, Val-GABOB, Leu-GABOB, Ile-GABOB, Pro-GABOB, Ser-GABOB, Thr-GABOB, Tyr-GABOB, Hyp-GABOB, Asp-GABOB, β-Asp-GABOB, D-Asp-GABOB, D-β-Asp-GABOB, Pyr-GABOB, Ala-EACA, Val-EACA, Leu-EACA, Ile-EACA, Pro-EACA, Ser-EACA, Thr-EACA, Hyp-EACA, Asp-EACA, β-Asp-EACA, D-Asp-EACA, D-β-Asp-EACA, Pyr-EACA,
γ-Glu-Gly-GABA, γ-Glu-Ala-GABA, γ-Glu-Val-GABA,
γ-Glu-Leu-GABA, γ-Glu-Ile-GABA, γ-Glu-Phe-GABA,
γ-Glu-Pro-GABA, γ-Glu-Ser-GABA, γ-Glu-Thr-GABA,
γ-Glu-Tyr-GABA, γ-Glu-Hyp-GABA, γ-Glu-Lys-GABA,
γ-Glu-His-GABA, γ-Glu-Arg-GABA, γ-Glu-Asp-GABA,
γ-Glu-Glu-GABA, γ-Glu-β-Asp-GABA,
γ-Glu-γ-Glu-GABA, γ-Glu-D-Asp-GABA,
γ-Glu-D-β-Asp-GABA, γ-Glu-Trp-GABA, γ-Glu-5-HTP-GABA, γ-Glu-Cys-GABA, γ-Glu-Met-GABA,
γ-Glu-Phe-β-Ala, γ-Glu-Phe-GABOB, γ-Glu-Phe-EACA, Glu-Phe-GABA, Glu-Phe-β-Ala, Glu-Phe-GABOB, Glu-Phe-EACA, Asp-Phe-GABA, Asp-Phe-β-Ala, Asp-Phe-GABOB, Asp-Phe-EACA, β-Asp-Phe-GABA, β-Asp-Phe-β-Ala, β-Asp-Phe-GABOB, β-Asp-Phe-EACA,

D-Asp-Phe-GABA, D-Asp-Phe-$\beta$-Ala, D-Asp-Phe-GABOB, D-Asp-Phe-EACA, D-$\beta$-Asp-Phe-GABA, D-$\beta$-Asp-Phe-$\beta$-Ala, D-ß-Asp-Phe-GABOB, D-$\beta$-Asp-Phe-EACA, $\gamma$-Glu-Pro-$\beta$-Ala, $\gamma$-Glu-Pro-GABOB, $\gamma$-Glu-Pro-EACA, Glu-Pro-GABA, Glu-Pro-$\beta$-Ala, Glu-Pro-GABOB, Glu-Pro-EACA, Asp-Pro-GABA, Asp-Pro-$\beta$-Ala, Asp-Pro-GABOB, Asp-Pro-EACA, $\beta$-Asp-Pro-GABA, $\beta$-Asp-Pro-$\beta$-Ala, .-Asp-Pro-GABOB, $\beta$-Asp-Pro-EACA, D-Asp-Pro-GABA, D-Asp-Pro-$\beta$-Ala, D-Asp-Pro-GABOB, D-Asp-Pro-EACA, D-$\beta$-Asp-Pro-GABA, D-$\beta$-Asp-Pro-$\beta$-Ala, D-$\beta$-Asp-Pro-GABOB, D-$\beta$-Asp-Pro-EACA, oder ein pharmazeutisch annehmbares Salz oder Metallkomplex davon.

## Revendications

## Revendications pour les Etats contractants suivants : AT, BE, CH, DE, FR, GB, IT, LI, LU, NL, SE

**1.** Composition pharmaceutique contenant comme principe actif un peptide ayant la formule (I)

A-X-NH-Y-CO-R

dans laquelle

A est l'hydrogène, un groupe amino-protecteur, un reste acide d'amino-acide, ou un reste acide d'amino-acide ayant un groupe protecteur, caractérisé en ce que :

i) quand A est l'hydrogène ou un groupe amino-protecteur :
- X est sélectionné parmi Ala, Val, Leu, Ile, Pro, Ser, Thr, Tyr, Hyp, Asp, D-Asp, $\beta$-Asp, D-$\beta$-Asp, Phe et Pyr, avec ou sans groupe protecteur;
- Y est un alkylène linéaire de 3 à 6 atomes de carbone ou un hydroxalkylène, et
- R est un groupe hydroxy ou un groupe carboxy protecteur; sous réserve d'exclure Pyr-GABA;

ii) quand A est un reste acide d'amino-acide ou un reste acide d'amino-acide ayant un groupe protecteur :
- X est un reste amino-acide avec ou sans groupe protecteur;
- Y est un alkylène linéaire de 2 à 6 atomes de carbones, ou un hydroxyalkylène, et
- R est un groupe hydroxy ou un groupe carboxy protecteur

ou un de ses sels ou complexes pharmaceutiquement acceptable.

**2.** Composition selon la revendication 1 (i) caractérisé en ce que Y est un groupe alkylène linéaire ayant 3 atomes de carbone.

**3.** Composition selon la revendication 1 (i) caractérisé en ce que Y est un groupe aklylène linéaire ayant 5 atomes de carbone.

**4.** Composition selon la revendication 1 (ii) caractérisé en ce que Y est un groupe alkylène linéaire ayant 2 atomes de carbone.

**5.** Composition selon la revendication 1 (ii) caractérisé en ce que Y est un groupe alkylène linéaire ayant 3 atomes de carbone.

**6.** Composition selon la revendication 1 (ii) caractérisé en ce que Y est un groupe alkylène linéaire ayant 5 atomes de carbone.

**7.** Composition selon la revendication 1 (ii) ou une quelconque des revendications 4 à 6, caractérisé en ce que X est un reste phénylalanine avec ou sans groupe protecteur.

**8.** Composition selon la revendication 1 (ii) ou une quelconque des revendications 4 à 6, caractérisé en ce que X est un reste proline avec ou sans groupe protecteur.

**9.** Composition selon une quelconque revendication précédente caractérisée en ce que X est un reste phénylalanine avec ou sans groupe protecteur.

**10.** Composition selon la revendication 1 contenant comme principe actif un peptide sélectionné parmi : Ala-GABA, Val-GABA, Leu-GABA, Ile-GABA, Phe-GABA, Pro-GABA, Ser-GABA, Thr-GABA, Tyr-GABA, Hyp-GABA, Asp-GABA, $\beta$-Asp-GABA, D-Asp-GABA, D-$\beta$-Asp-GABA,

Ala-GABOB, Val-GABOB, Leu-GABOB, Ile-GABOB, Phe-GABOB, Pro-GABOB, Ser-GABOB, Thr-GABOB, Tyr-GABOB, Hyp-GABOB, Asp-GABOB, β-Asp-GABOB, D-Asp-GABOB, D-β-Asp-GABOB, Pyr-GABOB,
Ala-EACA, Val-EACA, Leu-EACA, Ile-EACA, Phe-EACA, Pro-EACA, Ser-EACA, Thr-EACA, Hyp-EACA, Asp-EACA, β-Asp-EACA, D-Asp-EACA, D-β-Asp-EACA, Pyr-EACA,
γ-Glu-Gly-GABA, γ-Glu-Ala-GABA, γ-Glu-Val-GABA, γ-Glu-Leu-GABA, γ-Glu-Ile-GABA, γ-Glu-Phe-GABA, γ-Glu-Pro-GABA, γ-Glu-Ser-GABA, γ-Glu-Thr-GABA, γ-Glu-Tyr-GABA, γ-Glu-Hyp-GABA, γ-Glu-Lys-GABA, γ-Glu-His-GABA, γ-Glu-Arg-GABA, γ-Glu-Asp-GABA, γ-Glu-Glu-GABA, γ-Glu-β-Asp-GABA, γ-Glu-γ-Glu-GABA, γ-Glu-D-Asp-GABA, γ-Glu-D-β-Asp-GABA, γ-Glu-Trp-GABA, γ-Glu-5-HTP-GABA, γ-Glu-Cys-GABA, γ-Glu-Met-GABA, γ-Glu-Phe-β-Ala, γ-Glu-Phe-GABOB, γ-Glu-Phe-EACA,
Glu-Phe-GABA, Glu-Phe-β-Ala, Glu-Phe-GABOB, Glu-Phe-EACA,
Asp-Phe-GABA, Asp-Phe-β-Ala, Asp-Phe-GABOB, Asp-Phe-EACA,
β-Asp-Phe-GABA, β-Asp-Phe-β-Ala, β-Asp-Phe-GABOB, β-Asp-Phe-EACA, D-Asp-Phe-GABA, D-Asp-Phe-β-Ala, D-Asp-Phe-GABOB, D-Asp-Phe-EACA, D-β-Asp-Phe-GABA, D-β-Asp-Phe-β-Ala, D-β-Asp-Phe-GABOB, D-β-Asp-Phe-EACA,
γ-Glu-Pro-β-Ala, γ-Glu-Pro-GABOB, γ-Glu-Pro-EACA,
Glu-pro-GABA, Glu-Pro-β-Ala, Glu-Pro-GABOB, Glu-Pro-EACA,
Asp-Pro-GABA, Asp-Pro-β-Ala, Asp-Pro-GABOB, Asp-Pro-EACA,
β-Asp-Pro-GABA, β-Asp-Pro-β-Ala, β-Asp-Pro-GABOB, β-Asp-Pro-EACA,
D-Asp-Pro-GABA, D-Asp-Pro-β-Ala, D-Asp-Pro-GABOB, D-Asp-Pro-EACA,
D-β-Asp-Pro-GABA, D-β-Asp-Pro-β-Ala, D-β-Asp-Pro-GABOB, D-β-Asp-Pro-EACA,
ou un de ses sels ou complexes pharmaceutiquement acceptable.

**11.** Composition selon une quelconque revendication précédente possédant une activité neurotrope.

**12.** Composition neurotrope selon la revendication 11 possédant une activité sédative.

**13.** Composition neurotrope selon la revendication 11 possédant une activité métabolique cérébrale

**14.** Composition neurotrope selon la revendication 11 possédant une activité analgésique.

**15.** Composition neurotrope selon la revendication 11 comprenant Ala-GAB ou γ-Glu-Phe-GABA.

**16.** Application d'un peptide de la formule (I) tel que spécifié dans une quelconque des revendications 1 à 9 ou l'un de ses sels cou complexes pharmaceutiquement acceptable pour la fabrication d'agents possédant une activité sédative, métabolique cérébrale, ou analgésique.

**17.** Peptide ayant la formule (I)

A-X-NH-Y-CO-R

dans laquelle :

- A est l'hydrogène, un groupe amino-protecteur, un reste acide d'amino-acide, ou un reste acide d'amino-acide ayant un groupe protecteur, caractérisé en ce que :

i) quand A est l'hydrogène ou un groupe amino-protecteur :
  - X est sélectionné parmi Ala, Val, Leu, Ile, Pro, Ser, Thr, Tyr, Hyp, Asp, D-Asp, β-Asp, D-β-Asp et Pyr, avec ou sans groupe protecteur;
  - Y est un alkylène linéaire de 3 à 6 atome de carbone ou un hydroxalkylène, et
  - R est un groupe hydroxy ou un groupe carboxy protecteur; sous reserve d'exclure Pyr-GABA et Tyr-GABOB;

ii) Quand A est un reste acide d'amino-acide ou un reste acide d'amino-acide ayant un groupe protecteur :
  - X est un reste amino-acide avec ou sans groupe protecteur;
  - Y est un alkylène linéaire de 2 à 6 atomes de carbone, ou un hydroxalkylène, et
  - R est un groupe hydroxy ou un groupe carboxy protecteur

ou un sel ou complexe de celui-ci pharmaceutiquement acceptable.

**18.** Peptide, sel ou complexe selon la revendication 17 (i) caractérisé en ce que Y est un groupe alkylène linéaire ayant 3 atomes de carbone.

**19.** Peptide, sel ou complexe selon la revendication 17 (i) caractérisé en ce que Y est un groupe aklylène linéaire ayant 5 atomes de carbone.

**20.** Peptide, sel ou complexe selon la revendication 17 (ii) caractérisé en ce que Y est un groupe alkylène linéaire ayant 2 atomes de carbone.

**21.** Peptide, sel ou complexe selon la revendication 17 (ii) caractérisé en ce que Y est un groupe alkylène linéaire ayant 3 atomes de carbone.

**22.** Peptide, sel ou complexe selon la revendication 17 (ii) caractérisé en ce que Y est un goupe alkylène linéaire ayant 5 atomes de carbone.

**23.** Peptide, sel ou complexe selon la revendication 17 (ii) ou une quelconque des revendications 20 à 22, caractérisé en ce que X est un reste phénylalanine avec ou sans groupe protecteur.

**24.** Peptide, sel ou complexe selon revendication 17 (ii) ou une quelconque des revendications 4 à 6, caractérisé en ce que X est un reste proline avec ou sans groupe protecteur.

**25.** Ala-GABA, Val-GABA, Leu-GABA, Ile-GABA, Pro-GABA, Ser-GABA, Thr-GABA, Tyr-GABA, Hyp-GABA, Asp-GABA, $\beta$-Asp-GABA, D-Asp-GABA, D-$\beta$-Asp-GABA,
Ala-GABOB, Val-GABOB, Leu-GABOB, Ile-GABOB, Pro-GABOB, Ser-GABOB, Thr-GABOB, Tyr-GABOB, Hyp-GABOB, Asp-GABOB, $\beta$-Asp-GABOB, D-Asp-GABOB, D-$\beta$-Asp-GABOB, Pyr-GABOB,
Ala-EACA, Val-EACA, Leu-EACA, Ile-EACA, Pro-EACA, Ser-EACA, Thr-EACA, Hyp-EACA, Asp-EACA, $\beta$-Asp-EACA, D-Asp-EACA, D-$\beta$-Asp-EACA, Pyr-EACA,
$\gamma$-Glu-Gly-GABA, $\gamma$-Glu-Ala-GABA, $\gamma$-Glu-Val-GABA, $\gamma$-Glu-Leu-GABA, $\gamma$-Glu-Ile-GABA, $\gamma$-Glu-Phe-GABA, $\gamma$-Glu-Pro-GABA, $\gamma$-Glu-Ser-GABA, $\gamma$-Glu-Thr-GABA, $\gamma$-Glu-Tyr-GABA, $\gamma$-Glu-Hyp-GABA, $\gamma$-Glu-Lys-GABA, $\gamma$-Glu-His-GABA, $\gamma$-Glu-Arg-GABA, $\gamma$-Glu-Asp-GABA, $\gamma$-Glu-Glu-GABA, $\gamma$-Glu-$\beta$-Asp-GABA, $\gamma$-Glu-$\gamma$-Glu-GABA, $\gamma$-Glu-D-Asp-GABA, $\gamma$-Glu-D-$\beta$-Asp-GABA, $\gamma$-Glu-Trp-GABA, $\gamma$-Glu-5-HTP-GABA, $\gamma$-Glu-Cys-GABA, $\gamma$-Glu-Met-GABA, $\gamma$-Glu-Phe-$\beta$-Ala, $\gamma$-Glu-Phe-GABOB, $\gamma$-Glu-Phe-EACA,
Glu-Phe-GABA, Glu-Phe-$\beta$-Ala, Glu-Phe-GABOB, Glu-Phe-EACA,
Asp-Phe-GABA, Asp-Phe-$\beta$-Ala, Asp-Phe-GABOB, Asp-Phe-EACA,
$\beta$-Asp-Phe-GABA, $\beta$-Asp-Phe-$\beta$-Ala, $\beta$-Asp-Phe-GABOB, $\beta$-Asp-Phe-EACA, D-Asp-Phe-GABA, D-Asp-Phe-$\beta$-Ala, D-Asp-Phe-GABOB, D-Asp-Phe-EACA, D-$\beta$-Asp-Phe-GABA, D-$\beta$-Asp-Phe-$\beta$-Ala, D-$\beta$-Asp-Phe-GABOB, D-$\beta$-Asp-Phe-EACA,
$\gamma$-Glu-Pro-$\beta$-Ala, $\gamma$-Glu-Pro-GABOB, $\gamma$-Glu-Pro-EACA,
Glu-Pro-GABA, Glu-Pro-$\beta$-Ala, Glu-Pro-GABOB, Glu-Pro-EACA,
Asp-Pro-GABA, Asp-Pro-$\beta$-Ala, Asp-Pro-GABOB, Asp-Pro-EACA,
$\beta$-Asp-Pro-GABA, $\beta$-Asp-Pro-$\beta$-Ala, $\beta$-Asp-Pro-GABOB, $\beta$-Asp-Pro-EACA,
D-Asp-Pro-GABA, D-Asp-Pro-$\beta$-Ala, D-Asp-Pro-GABOB, D-Asp-Pro-EACA,
D-$\beta$-Asp-Pro-GABA, D-$\beta$-Asp-Pro-$\beta$-Ala, D-$\beta$-Asp-Pro-GABOB, D-$\beta$-Asp-Pro-EACA,

**26.** Procédé de préparation d'un peptide selon la revendication 17 comprenant les étapes de couplage d'un ou des peptides partiels, ou d'un ou des amino-acides avec le résidu restant correspondant pour constituer le composé de formule (I) et éliminer si nécessaire tout groupe protecteur.

**Revendications pour les Etats contractants suivants : ES, GR**

**1.** Procédé pour la préparation d'un péptide ayant la formule (I)

A-X-NH-Y-CO-R

dans laquelle
- A est l'hydrogène, un groupe amino-protecteur, un reste acide d'amino-acide, ou un reste acide d'amino-acide ayant un groupe protecteur, caractérisé en ce que :

i) quand A est l'hydrogène ou un groupe amino-protecteur :

- X est sélectionné parmi Ala, Val, Leu, Ile, Pro, Ser, Thr, Tyr, Hyp, Asp, D-Asp, β-Asp, D-β-Asp et Pyr, avec ou sans groupe protecteur;
- Y est un alkylène linéaire de 3 à 6 atomes de carbone ou un hydroxalkylène, et
- R est un groupe hydroxy ou un groupe carboxy protecteur; sous reserve d'exclure Pyr-GABA et Tyr-GABOB;

ii) Quand A est un reste acide d'amino-acide ou un reste acide d'amino-acide ayant un groupe protecteur :

- X est un reste amino-acide avec ou sans groupe protecteur;
- Y est un alkylène linéaire de 2 à 6 atomes de carbone, ou un hydroxyalkylène, et
- R est un groupe hydroxy ou un groupe carboxy protecteur ou un sel ou complexe de celui-ci pharmaceutiquement acceptable comprenant les étapes de couplage d'un ou des peptides partiels, ou d'un ou des amino-acides avec le résidu restant correspondant pour constituer le composé de formule (I) et éliminer si nécessaire tout groupe protecteur.

**2.** Procédé selon la revendication 1 (i) caractérisé en ce que Y est un groupe alkylène linéaire ayant 3 atomes de carbone.

**3.** Procédé selon la revendication 1 (i) caractérisé en ce que Y est un groupe aklylène linéaire ayant 5 atomes de carbone.

**4.** Procédé selon la revendication 1 (ii) caractérisé en ce que Y est un groupe alkylène linéaire ayant 2 atomes de carbone.

**5.** Procédé selon la revendication 1 (ii) caractérisé en ce que Y est un groupe alkylène linéaire ayant 3 atomes de carbone.

**6.** Procédé selon la revendication 1 (ii) caractérisé en ce que Y est un groupe alkylène linéaire ayant 5 atomes de carbone.

**7.** Procédé selon la revendication 1 (ii) ou une quelconque des revendications 2 à 4, caractérisé en ce que X est un reste phénylalanine avec ou sans groupe protecteur.

**8.** Procédé selon revendication 1 (ii) ou une quelconque des revendications 2 à 4, caractérisé en ce que X est un reste proline avec ou sans groupe protecteur.

**9.** Procédé selon la revendication 1 caractérisé en ce que le peptide est :
Ala-GABA, Val-GABA, Leu-GABA, Ile-GABA, Pro-GABA, Ser-GABA, Thr-GABA, Tyr-GABA, Hyp-GABA, Asp-GABA, β-Asp-GABA, D-Asp-GABA, D-β-Asp-GABA,
Ala-GABOB, Val-GABOB, Leu-GABOB, Ile-GABOB, Pro-GABOB, Ser-GABOB, Thr-GABOB, Tyr-GABOB, Hyp-GABOB, Asp-GABOB, β-Asp-GABOB, D-Asp-GABOB, D-β-Asp-GABOB, Pyr-GABOB,
Ala-EACA, Val-EACA, Leu-EACA, Ile-EACA, Pro-EACA, Ser-EACA, Thr-EACA, Hyp-EACA, Asp-EACA, β-Asp-EACA, D-Asp-EACA, D-β-Asp-EACA, Pyr-EACA,
γ-Glu-Gly-GABA, γ-Glu-Ala-GABA, γ-Glu-Val-GABA, γ-Glu-Leu-GABA, γ-Glu-Ile-GABA, γ-Glu-Phe-GABA, γ-Glu-Pro-GABA, γ-Glu-Ser-GABA, γ-Glu-Thr-GABA, γ-Glu-Tyr-GABA, γ-Glu-Hyp-GABA, γ-Glu-Lys-GABA, γ-Glu-His-GABA, γ-Glu-Arg-GABA, γ-Glu-Asp-GABA, γ-Glu-Glu-GABA, γ-Glu-β-Asp-GABA, γ-Glu-γ-Glu-GABA, γ-Glu-D-Asp-GABA, γ-Glu-D-β-Asp-GABA, γ-Glu-Trp-GABA, γ-Glu-5-HTP-GABA, γ-Glu-Cys-GABA, γ-Glu-Met-GABA, γ-Glu-Phe-β-Ala, γ-Glu-Phe-GABOB, γ-Glu-Phe-EACA,
Glu-Phe-GABA, Glu-Phe-β-Ala, Glu-Phe-GABOB, Glu-Phe-EACA,
Asp-Phe-GABA, Asp-Phe-β-Ala, Asp-Phe-GABOB, Asp-Phe-EACA,
β-Asp-Phe-GABA, β-Asp-Phe-β-Ala, β-Asp-Phe-GABOB, β-Asp-Phe-EACA, D-Asp-Phe-GABA, D-Asp-Phe-β-Ala, D-Asp-Phe-GABOB, D-Asp-Phe-EACA, D-β-Asp-Phe-GABA, D-β-Asp-Phe-β-Ala, D-β-Asp-Phe-GABOB, D-β-Asp-Phe-EACA,
γ-Glu-Pro-β-Ala, γ-Glu-Pro-GABOB, γ-Glu-Pro-EACA,
Glu-Pro-GABA, Glu-Pro-β-Ala, Glu-Pro-GABOB, Glu-Pro-EACA,
Asp-Pro-GABA, Asp-Pro-β-Ala, Asp-Pro-GABOB, Asp-Pro-EACA,
β-Asp-Pro-GABA, β-Asp-Pro-β-Ala, β-Asp-Pro-GABOB, β-Asp-Pro-EACA,
D-Asp-Pro-GABA, D-Asp-Pro-β-Ala, D-Asp-Pro-GABOB, D-Asp-Pro-EACA,
D-β-Asp-Pro-GABA, D-β-Asp-Pro-β-Ala, D-β-Asp-Pro-GABOB,

D-β-Asp-Pro-EACA,
ou un de ses sels ou complexes pharmaceutiquement acceptable.